# EUROPEAN PATENT APPLICATION

(11) **EP 4 647 421 A1**
(43) Date of publication of application: **12.11.2025**
(21) Application number: 23914168.2
(22) Date of filing: 29.03.2023
(51) Int. Cl.: C07C 275/14, A61K 31/7088, A61K 31/7105, A61P 35/00, A61P 31/12, A61P 31/14, A61K 47/18, A61K 9/127

(54) **COMPOUND FOR DELIVERING DRUG, AND LIPOSOME AND DRUG CARRIER**

(30) Priority: 04.01.2023 CN 202310009545
(71) Applicant: Westgene Biopharma Co., Ltd., Chengdu, Sichuan 610000 (CN)
(72) Inventor: SONG, Xiangrong, Chengdu, Sichuan 610000 (CN); HUANG, Hai, Chengdu, Sichuan 610000 (CN); WEI, Xiawei, Chengdu, Sichuan 610000 (CN); WEI, Yuquan, Chengdu, Sichuan 610000 (CN)
(74) Representative: karo IP
(86) International application number: PCT/CN2023/084757
(87) International publication number: WO 2024/146016

(57) **Abstract**

The present invention provides a compound, which is a compound represented by formula (I) or a stereoisomer, tautomer, solvate, or pharmaceutically acceptable salt thereof represented by formula (I): 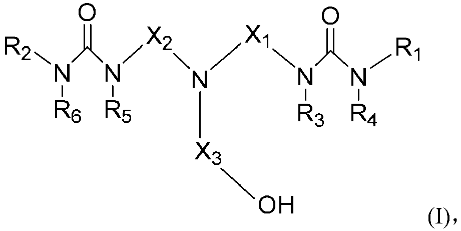 X1, X2, and X3 are each independently a C1-C15 alkyl group optionally substituted; R1 and R2 are each independently a C1-C40 alkyl group optionally substituted, a C1-C40 heteroalkyl group optionally substituted, a C2-C40 alkenyl group optionally substituted, a C2-C40 heteroalkenyl group optionally substituted, a C2-C40 alkynyl group optionally substituted, or a C2-C40 heteroalkynyl group optionally substituted; R3, R4, R5, and R6 are each independently H, halogen, or a C1-C3 alkyl group optionally substituted; the substituent group is independently selected from halogen, -OH, -SH, -NH2, -NO2, cyano, or C1-C3 alkyl. This compound has the advantages of low cytotoxicity, strong delivery capability, and good immunostimulatory effect.

## Description

### Technical Field

This invention belongs to the field of biotechnology. Specifically, it pertains to a compound for drug delivery, liposomes, and drug carriers. More specifically, it involves a compound and its uses, liposomes, drug carriers, complexes, pharmaceutical compositions, and their pharmaceutical applications.

### Background Technology

Gene therapy technology is a hot research topic in the modern biomedical field. Nucleic acid drugs, such as small interfering RNA (siRNA), messenger RNA (mRNA), and plasmid DNA (pDNA), can be used to prevent cancers, bacterial and viral infections, and treat diseases with genetic causes. Due to the characteristics of nucleic acid drugs, such as easy degradation and difficulty in entering cells, carriers are needed to encapsulate and deliver them to target cells, making the development of safe and efficient delivery carriers a prerequisite for the clinical application of gene therapy.

Currently, the most common nucleic acid delivery systems are two types of carriers: viral carriers and non-viral carriers. Viral carriers have relatively high transfection efficiency but pose safety risks and lack target specificity. Liposomes, as representatives of non-viral carriers, have developed rapidly due to their low immunogenicity, good biocompatibility, and high transfection efficiency, and are considered ideal nucleic acid delivery systems. However, traditional lipid compound nucleic acid delivery systems suffer from low efficiency, high toxicity, and poor targeting.

Among non-viral carriers used for nucleic acid delivery, LNPs (Lipid Nanoparticles) have shown outstanding performance and have been used in three approved nucleic acid drugs, including Patisiran (an siRNA drug, commercial name: ONPATTRO), BNT162b2, and mRNA-1273. LNPs typically consist of four components: ionizable lipids, neutral phospholipids, cholesterol, and polyethylene glycol-modified lipids. Ionizable lipids play a key role in LNPs, determining mRNA loading, expression levels, and targeting, as evidenced by the ionizable lipids used in Patisiran, BNT162b2, and mRNA-1273, which are DLin-MC3-DMA (MC3), ALC-0315, and SM-102, respectively. The LNP delivery platform, as a very promising mRNA drug delivery vehicle, has attracted significant attention in the pharmaceutical industry and is continuously expanding into other research fields. However, the intrinsic mechanisms of efficient mRNA delivery by ionizable lipid LNPs remain to be elucidated. The principle of mRNA loading in LNPs is based on electrostatic adsorption; despite similar encapsulation rates, the in vivo expression levels of LNP-mRNA can vary, with the only variable being the chemical structure of the ionizable lipid. Moreover, the chemical structure of the ionizable lipid also affects the distribution of mRNA expression in vivo. As the range of applications for mRNA drugs expands, targeting different diseases and targets will place higher demands on the delivery system, making targeted delivery of mRNA an urgent scientific problem to solve.

Thus, there is a pressing need to develop a lipid compound with high efficiency, low toxicity, and excellent targeting capabilities.

### Content of the Invention

This invention aims to address at least one of the technical problems existing in the prior art, at least to a certain extent. To this end, the invention provides a compound for drug delivery, which exhibits low cytotoxicity, strong delivery capabilities, and effective immune activation.

In one aspect of the invention, a compound is proposed, which is the compound represented by formula (I), or a stereoisomer, tautomer, solvate, or pharmaceutically acceptable salt of the compound represented by formula (I):

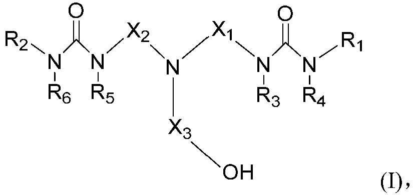

wherein X₁, X₂, and X₃ are each independently selected from optionally substituted C₁-C₁₅ alkyl groups; R₁ and R₂ are each independently selected from optionally substituted C1-C40 alkyl, optionally substituted C₁-C₄₀ heteroalkyl, optionally substituted C₂-C₄₀ alkenyl, optionally substituted C₂-C₄₀ heteroalkenyl, optionally substituted C₂-C₄₀ alkynyl, or optionally substituted C₂-C₄₀ heteroalkynyl; R₃, R₄, R₅, and R₆ are each independently selected from H, halogen, or optionally substituted C₁-C₃ alkyl; wherein the substituent groups are independently selected from one or more of halogen, -OH, -SH, -NH₂, -NO₂, cyanide, or C₁-C₃ alkyl. The compounds of the invention exhibit low cytotoxicity, showing good biocompatibility; these compounds have strong delivery capabilities, serving as delivery carriers that can transport nucleic acid drugs to organs such as the heart, liver, spleen, lungs, and kidneys, particularly effective in delivering to and activating the immune system in the spleen, thereby enhancing the levels of specific antibodies within the animal body.

In another aspect of the invention, a use of the aforementioned compound in the preparation of liposomes, drug carriers, or complexes is proposed. According to an embodiment of the invention, the aforementioned compound has ionizable properties and can be used to prepare liposomes. The liposomes prepared can serve as drug carriers, forming complexes with nucleic acid drugs.

In yet another aspect of the invention, a liposome is proposed. According to an embodiment of the invention, the liposome includes: the aforementioned compound. The liposome according to the embodiment of the invention has low cytotoxicity, good biocompatibility, strong delivery capabilities, and effective immune activation.

In another aspect of the invention, a liposome is proposed. According to an embodiment of the invention, the liposome includes the aforementioned compound, neutral lipids, cholesterol, and PEG-lipids. The neutral lipids include those selected from 1,2-dioleoyl-sn-glycerol-3-phosphate ethanolamine (DOPE) and/or 1,2-dioleoyl-sn-glycerol-3-phosphocholine (DOPC). The PEG-lipid is 1,2-dimyristoyl-sn-glycerol methoxy polyethylene glycol (PEG-DMG). The molar ratio of the compound, neutral lipids, cholesterol, and PEG-lipids is (40~60):(5~10):(30~50):(0.5~5).

In another aspect of the invention, a drug carrier is proposed. According to an embodiment of the invention, the drug carrier includes the aforementioned compound or the aforementioned liposome. According to the embodiment of the invention, the aforementioned compound is an ionizable lipid, and the drug carrier is an ionizable carrier, which uses the aforementioned compound or liposome to load drugs and deliver them into cells.

In another aspect of the invention, a complex is proposed. According to an embodiment of the invention, the complex includes: the aforementioned compound, the aforementioned liposome, or the aforementioned drug carrier; and a biologically active ingredient. As previously stated, the aforementioned compound is an ionizable lipid, and the drug carrier is an ionizable carrier. Therefore, using the aforementioned compound or liposome to load drugs can prepare a complex containing biologically active ingredients, which can deliver biologically active ingredients into the cells of an organism for the treatment of diseases.

In another aspect of the invention, a pharmaceutical composition is proposed. According to an embodiment of the invention, the pharmaceutical composition includes: the aforementioned compound, the aforementioned liposome, the aforementioned drug carrier, or the aforementioned complex. As previously mentioned, these components exhibit low cytotoxicity, good biocompatibility, strong delivery capabilities, and effective immune activation. By loading biological active ingredients into the aforementioned compound, liposome, or drug carrier, these active ingredients can be delivered within the organism, facilitating the therapeutic effects of the loaded bioactive components for the treatment of diseases.

In another aspect of the invention, a use of the aforementioned compound, the aforementioned liposome, the aforementioned drug carrier, the aforementioned complex, or the aforementioned pharmaceutical composition in the preparation of a drug is proposed. The drug is intended for targeting at least one of the heart, liver, spleen, lungs, and kidneys. As discussed earlier, due to their low cytotoxicity, good biocompatibility, strong delivery capabilities, and effective immune activation, these components, when loaded with biological active ingredients, can deliver these components to the heart, liver, spleen, lungs, and kidneys, benefiting the therapeutic effects of the loaded bioactive components for the treatment of diseases.

Furthermore, another aspect of the invention proposes the use of the aforementioned compound, the aforementioned liposome, the aforementioned drug carrier, the aforementioned complex, or the aforementioned pharmaceutical composition in the preparation of a drug for the treatment or prevention of diseases. As previously described, these components have low cytotoxicity, good biocompatibility, strong delivery capabilities, and effective immune activation. By loading them with biological active ingredients, these ingredients can be delivered within the organism, facilitating their therapeutic efficacy for disease treatment.

Additional aspects and advantages of the invention will be set forth in part in the description that follows, and in part will become apparent from the description, or may be learned by practice of the invention.

### Figure Descriptions

The aforementioned and/or additional aspects and advantages of the invention will become apparent and readily understood through the following descriptions of the embodiments in conjunction with the accompanying drawings, where:
Figure 1 shows the results of cytotoxicity evaluation for different compounds used in Test Example step 1 of the invention.
Figure 2 presents images of the bioluminescence intensity in various dissected organs of mice injected with different LNPs@FLuc mRNA drugs, as part of Test Example step 2.
Figure 3 displays the total fluorescence statistics for various dissected organs of mice injected with different LNPs@FLuc mRNA drugs, as part of Test Example step 2.
Figure 4 shows the results of antibody titers in the serum of mice injected with different LNPs@FLuc mRNA drugs, included in Test Example step 3.
Figure 5 is the H1-NMR spectrum of Compound 7 of the invention.
Figure 6 is the H1-NMR spectrum of Compound 8 of the invention.
Figure 7 is the H1-NMR spectrum of Compound 9 of the invention.
Figure 8 is the H1-NMR spectrum of Compound 10 of the invention.
Figure 9 is the H1-NMR spectrum of Compound 11 of the invention.
Figure 10 is the H1-NMR spectrum of Compound 12 of the invention.
Figure 11 is the H1-NMR spectrum of Compound 13 of the invention.
Figure 12 is the H1-NMR spectrum of Compound 15 of the invention.
Figure 13 is the H1-NMR spectrum of Compound 16 of the invention.
Figure 14 is the H1-NMR spectrum of Compound 17 of the invention.
Figure 15 is the H1-NMR spectrum of Compound 18 of the invention.
Figure 16 is the H1-NMR spectrum of Compound 19 of the invention.
Figure 17 is the H1-NMR spectrum of Compound 21 of the invention.

### Detailed Embodiments

The embodiments of this invention are described in detail below. These embodiments are illustrative and are intended to explain the invention without being restrictive.

It should be noted that the terms "first," "second," etc., are used only for descriptive purposes and should not be interpreted as indicating relative importance or implicitly specifying the number of technical features indicated. Thus, the features defined as "first," "second," may explicitly or implicitly include one or more of such features. Moreover, in the description of the present invention, unless otherwise specified, "multiple" means two or more.

It should also be noted that in the context of the structural and chemical formulae described in the embodiments or implementation schemes of this invention, the intention is to cover all alternatives, modifications, and equivalents that fall within the scope of the invention as defined by the claims. Persons skilled in the relevant field should recognize that many methods and materials similar to or equivalent to those described in relation to this invention can be used to implement the invention. This invention is not limited to the methods and materials described herein. In case there is any discrepancy or inconsistency in terms or technology described between this application and the literature, patents, or similar materials combined herein (including but not limited to defined terms, applications of terms, described technologies, etc.), the details in this invention prevail.

It should further be recognized that certain features of the invention, for clarity, are described across multiple independent embodiments or implementation schemes, but they may also be provided in a combined form in a single embodiment or implementation scheme. Conversely, various features of the invention, for the sake of brevity, are described in a single embodiment or implementation scheme but can also be provided individually or in any suitable subcombination.

Unless otherwise specified, the technical and scientific terms used in this invention have the same meaning as commonly understood by those of ordinary skill in the art to which this invention belongs, unless defined otherwise. All patents, publications, and other public disclosures referred to or cited in this disclosure are incorporated herein by reference in their entirety.

For the purposes of this invention, chemical elements are defined according to the periodic table from the CAS version and the Chemical Abstracts Service's Chemical Handbook, 75th Edition, 1994. Additionally, general principles of organic chemistry can be referred to in "Organic Chemistry" by Thomas Sorrell, University Science Books, Sausalito: 1999, and "March's Advanced Organic Chemistry" by Michael B. Smith and Jerry March, John Wiley & Sons, New York: 2007, thus all contents of these references are incorporated into this invention.

In this document, the terms "comprising" or "including" are used in an open-ended manner, meaning they encompass the elements specified by the invention but do not exclude other elements.

In this document, the compounds of the invention also include isotopically labeled versions of the compounds of the invention, which are identical to those described in the invention except for the fact that one or more atoms are replaced by isotopes with atomic masses or mass numbers different from the naturally occurring atomic masses or mass numbers. Exemplary isotopes that can be introduced into the compounds of the invention include isotopes of hydrogen, carbon, nitrogen, oxygen, phosphorus, sulfur, fluorine, and chlorine, such as ²H, ³H, ¹³C, ¹⁴C, ¹⁵N, ¹⁶O, ¹⁷O, ³¹P, ³²P, ³⁶S, ¹⁸F, and ³⁷Cl.

The compounds of the invention that contain the aforementioned isotopes and/or isotopes of other atoms, as well as pharmaceutically acceptable salts of said compounds, are included within the scope of this invention. Isotopically labeled compounds of the invention, such as radioactive isotopes like ³H and ¹⁴C, can be incorporated into the compounds of the invention for use in the analysis of drug and/or substrate tissue distribution. Due to their ease of preparation and detection, tritium (³H) and carbon-14 (¹⁴C) isotopes are particularly preferred. Additionally, replacement with heavy isotopes, such as deuterium (²H), can provide therapeutic advantages stemming from greater metabolic stability, such as increased in vivo half-life or reduced dosage requirements. Therefore, in some cases, this may be preferred.

The stereochemical definitions and conventions used in this invention largely follow those outlined in S.P. Parker, Ed., *McGraw-Hill Dictionary of Chemical Terms* (1984) McGraw-Hill Book Company, New York; and Eliel, E. and Wilen, S., *Stereochemistry of Organic Compounds*, John Wiley & Sons, Inc., New York, 1994. The compounds of this invention may contain asymmetric or chiral centers, thus existing in different stereoisomeric forms. It is anticipated that all stereoisomeric forms of the compounds of the invention, including but not limited to enantiomers, diastereomers, and atropisomers, as well as their mixtures such as racemic mixtures, are encompassed within the scope of this invention.Many organic compounds exist in optically active forms, meaning they have the ability to rotate the plane of plane-polarized light. When describing compounds that are optically active, prefixes D and L or R and S are used to denote the absolute configuration of the molecule with respect to its chiral center(s). The prefixes d and 1 or (+) and (-) are used to specify the sign of the rotation of plane-polarized light caused by the compound, where (-) or 1 indicates that the compound is levorotatory (rotates light to the left). Compounds prefixed with (+) or d are dextrorotatory (rotate light to the right). For a given chemical structure, aside from these stereoisomers being mirror images of each other, they are identical. Specific stereoisomers can also be referred to as enantiomers, and mixtures of these isomers are typically called racemic mixtures. A 50:50 mixture of enantiomers is known as a racemic mixture or racemate, which can occur when there is no stereo selectivity or stereo specificity in a chemical reaction or process.

Based on the choice of raw materials and methods, the compounds of this invention can exist in one or more possible isomeric forms, such as pure enantiomers or as mixtures of isomers, including racemic and non-corresponding diastereomeric mixtures, depending on the number of asymmetric carbon atoms. Optically active (R)- or (S)-isomers can be prepared using chiral synthons or chiral agents, or resolved using conventional techniques. If the compound contains a double bond, the substituents may be in E or Z configurations; if the compound includes a disubstituted cycloalkane, the substituents on the cycloalkane may be in cis or trans configurations.

The compounds of this invention may contain asymmetric or chiral centers and, therefore, exist in various stereoisomeric forms. It is anticipated that all stereoisomeric forms of the compounds of this invention, including but not limited to enantiomers, diastereomers, atropisomers, and geometric (or conformational) isomers, as well as their mixtures such as racemic mixtures, are encompassed within the scope of this invention.

Unless otherwise indicated, the structures described in this invention also include all isomeric forms of these structures (e.g., enantiomers, diastereomers, atropisomers, and geometric or conformational isomers); for example, R and S configurations at each asymmetric center, (Z) and (E) double bond isomers, and (Z) and (E) conformational isomers. Thus, individual stereoisomers of this invention, as well as mixtures of enantiomers, mixtures of diastereomers, and mixtures of geometric (or conformational) isomers, are all within the scope of this invention.

The compounds of this invention can exist in various stereoisomeric forms due to their asymmetric atoms (such as carbon). For instance, these atoms may be present in racemic or enantiomerically enriched forms, such as in (R)-, (S)-, or (R, S)- configurations. In certain embodiments, each asymmetric atom may possess an enantiomeric excess of at least 50%, at least 60%, at least 70%, at least 80%, at least 90%, at least 95%, or at least 99%. If applicable, substituents on atoms with unsaturated double bonds can exist in either cis-(Z)- or trans-(E)-configurations.

As described in this invention, the compounds can exist as any possible isomer, rotamer, atropisomer, tautomer, or as mixtures of these, such as substantially pure geometric (cis or trans) isomers, diastereomers, optical isomers (enantiomers), racemates, or their mixtures. The resulting mixtures of any isomers can be separated into pure or substantially pure geometric or optical isomers, diastereomers, or racemates based on the physicochemical differences of the components, for instance, by chromatography and/or fractional crystallization.

Any mixture of isomers obtained can be separated into pure or substantially pure geometric or optical isomers, diastereomers, or racemates based on their physicochemical differences, using methods such as chromatography and/or fractional crystallization.

Racemic mixtures of any final product or intermediate can be resolved into optical enantiomers using methods familiar to those skilled in the art. For example, separation can be achieved by isolating salts of the non-racemic diastereomers. Racemic products can also be separated by chiral chromatography, such as high-performance liquid chromatography (HPLC) using a chiral adsorbent. Specifically, enantiomers can be prepared through asymmetric synthesis, as outlined in sources like Jacques, et al., "Enantiomers, Racemates and Resolutions" (Wiley Interscience, New York, 1981); "Principles of Asymmetric Synthesis" (2nd Ed. Robert E. Gawley, Jeffrey Aubé, Elsevier, Oxford, UK, 2012); Eliel, E.L. "Stereochemistry of Carbon Compounds" (McGraw-Hill, NY, 1962); and Wilen, S.H. "Tables of Resolving Agents and Optical Resolutions" p. 268 (E.L. Eliel, Ed., University of Notre Dame Press, Notre Dame, IN 1972).

In this document, the terms "tautomer" or "tautomeric form" refer to structural isomers with different energies that can interconvert via a low energy barrier. If tautomerism is possible (such as in solution), a chemical equilibrium of the tautomeric forms can be achieved. For example, prototropic tautomers (also known as proton tautomers) involve interconversion through proton transfer, such as keto-enol tautomerism and imine-enamine tautomerism. Valence tautomers involve interconversion through the rearrangement of some bonding electrons. A specific example of keto-enol tautomerism is the interconversion between pentane-2,4-dione and 4-hydroxypent-3-en-2-one. Another example of tautomerism is the phenol-ketone tautomerism. A specific instance of phenol-ketone tautomerism is the interconversion between pyridin-4-ol and pyridin-4(1H)-one. Unless otherwise noted, all tautomeric forms of the compounds of this invention are included within the scope of this invention.

In this document, the term "solvate" refers to a complex formed by one or more solvent molecules with a compound of this invention. Solvents that can form solvates include, but are not limited to, water, isopropanol, ethanol, methanol, dimethyl sulfoxide, ethyl acetate, acetic acid, and ethanolamine. The term "hydrate" specifically refers to a complex where the solvent molecule is water.

The term "pharmaceutically acceptable" refers to substances or compositions that must be chemically and/or toxicologically compatible with the other components of the formulation and/or with the mammal being treated therapeutically.

In this document, the term "pharmaceutically acceptable salts" refers to organic and inorganic salts of the compounds of this invention. Pharmaceutically acceptable salts are well known in the field, as described by S. M. Berge et al., in J. Pharmaceutical Sciences, 1977, 66: 1-19. Pharmaceutically acceptable nontoxic acid salts include but are not limited to, inorganic acid salts formed with amino groups (such as hydrochloride, hydrobromide, phosphate, sulfate, and perchlorate) and organic acid salts (such as acetate, oxalate, malate, tartrate, citrate, succinate, and glutarate), or obtained by other methods like ion exchange documented in the literature. Other pharmaceutically acceptable salts include adipate, alginate, ascorbate, aspartate, benzenesulfonate, benzoate, bisulfate, borate, butyrate, camphorsulfonate, camphorate, caprylate, digluconate, dodecyl sulfate, ethanesulfonate, formate, fumarate, glucoheptonate, glycerophosphate, gluconate, half sulfate, heptanoate, hexanoate, hydroiodide, 2-hydroxyethanesulfonate, lactobionate, lactate, laurate, lauryl sulfate, malate, maleate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, tosylate, undecanoate, valerate, and others. Salts derived from suitable bases include those of alkali metals, alkaline earth metals, ammonium, and N⁺(C₁-C₄ alkyl)₄. This invention also contemplates the quaternary ammonium salts of any compounds containing an N-containing group. Water-soluble or oil-soluble or dispersible products can be obtained by quaternization. Alkali metal or alkaline earth metal salts include sodium, lithium, potassium, calcium, magnesium, and others. Pharmaceutically acceptable salts further include appropriate, nontoxic amine cations and quaternary ammonium cations and counterions such as halides, hydroxides, carboxylates, sulfates, phosphates, nitrates, C₁-C₈ sulfonates, and aromatic sulfonates.

In this document, the terms "optionally," and "optional"generally indicate that the subsequent event or condition may or may not occur, and the description includes scenarios where the event or condition occurs, as well as scenarios where it does not occur.

In this document, the terms "optionally substituted" and "substituted or unsubstituted" can be used interchangeably. Generally, the term "optionally," whether placed before the term "substituted" or not, indicates that one or more hydrogen atoms in the given structure may be replaced by a specific substituent. Unless indicated otherwise, an optional substituent group can be substituted at any permissible position on the group. When more than one position in a given structural formula can be substituted by one or more substituents from a specific group, the substituents can be the same or different at each position. The mentioned substituents can include, but are not limited to, F, Cl, Br, CN, OH, NH₂, NO₂, etc.

In this document, the term "one or more" (for example, in the definition of substituents in the general formula of the compounds of the invention) refers to "one, two, three, four, or five, especially one, two, three, or four, more particularly one, two, or three, and most particularly one or two."

In this invention, the phrases "each independently," "each independently of," and "independently" are used interchangeably and should be understood broadly. These terms indicate that the choices or options expressed between the same symbols within different groups, or within the same group, do not affect each other. This means that each component or substituent within a group can be selected without regard to the choices made for other components or substituents within the same group or in different groups.

In this document, the term "halogen" refers to fluorine, chlorine, bromine, or iodine atoms.

In this document, the range of carbon atoms in a hydrocarbon group is indicated by prefixes that specify the minimum and maximum number of carbon atoms. For example, the prefix "Cₐ-C_{b}" denotes a range that includes carbon atoms from "a" to "b". Illustratively, "C₁-Cₙ" refers to a hydrocarbon chain that contains 1, 2, 3, 4, 5, ..., up to n carbon atoms. This chain can be straight or branched and can be either saturated or unsaturated.

Further, it is understood that "C1-Cn" should be interpreted to include any sub-range within it. For example, the ranges C₁-C₄₀, C₂-C₄₀, C₁-C₂₄, C₃-C₂₄, C₁-C₁₁, C₄-C₁₀, C₄-C₈, and C₁-C₃ are all considered part of the broader "C₁-Cₙ" designation. This notation provides flexibility in specifying the size and structure of hydrocarbon groups within chemical compounds, adapting to various chemical and physical properties required in their applications.

In this document, the term "C₁-C₄₀ alkyl" refers to saturated monovalent hydrocarbon groups with 1 to 40 carbon atoms, which may be straight-chain or branched. Examples include C₂-C₄₀ alkyl, C₂-C₂₄ alkyl, C₃-C₂₄ alkyl, C₃-C₁₁ alkyl, C₄-C₁₀ alkyl, and C₄-C₈ alkyl. Specific examples of such alkyl groups include methyl, ethyl, n-propyl (n-Pr, -CH₂CH₂CH₃), isopropyl (i-Pr, -CH(CH₃)₂), n-butyl (n-Bu, -CH₂CH₂CH₂CH₃), isobutyl (i-Bu, -CH₂CH(CH₃)₂), sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, etc. These alkyl groups may be unsubstituted or optionally substituted with one or more substituents as described in this invention.

In this document, the term "C₂-C₄₀ alkenyl" refers to a monovalent hydrocarbon group containing 2 to 40 carbon atoms, which may be straight-chain or branched, with at least one position in the carbon-carbon structure being an sp2 double bond, making it unsaturated. These alkenyl groups may be unsubstituted or optionally substituted with one or more substituents as described in this invention. Additionally, the groups may have "cis" or "trans" (or "Z" or "E") configurations where applicable. Specific examples include, but are not limited to, vinyl (-CH=CH₂), allyl (-CH₂CH=CH₂), etc. Examples of ranges include C₂-C₄₀ alkenyl, C₂-C₂₄ alkenyl, C₃-C₂₄ alkenyl, C₃-C₁₁ alkenyl, C₄-C₁₀ alkenyl, and C₄-C₈ alkenyl.

In this document, the term "C₂-C₄₀ alkynyl" refers to a monovalent hydrocarbon group containing 2 to 40 carbon atoms, which may be straight-chain or branched, with at least one carbon-carbon triple bond, indicating an sp unsaturation. These alkynyl groups may be unsubstituted or optionally substituted with one or more substituents as described in this invention. Specific examples include but are not limited to ethynyl (-C≡CH), propargyl (-CH₂C≡CH), and 1-propynyl (-C≡C-CH₃). Ranges include C₂-C₄₀ alkynyl, C₂-C₂₄ alkynyl, C₃-C₂₄ alkynyl, C₃-C₁₁ alkynyl, C₄-C₁₀ alkynyl, and C₄-C₈ alkynyl.

The term "heteroalkyl" refers to alkyl groups that may include heteroatoms such as oxygen, sulfur, phosphorus, and nitrogen (in the form of a tertiary amine), providing heteroalkyl groups (e.g., alkyl containing one or more ether, thioether, or amine bonds). The term "heteroalkenyl" refers to alkenyl groups that may include heteroatoms, providing heteroalkenyl groups (e.g., alkenyl containing one or more ether, thioether, or amine bonds). The term "heteroalkynyl" refers to alkynyl groups that may include heteroatoms, providing heteroalkynyl groups (e.g., alkynyl containing one or more ether, thioether, or amine bonds). These definitions include modifications where the basic hydrocarbon skeleton is altered by the inclusion of these heteroatoms, which can affect the chemical and physical properties of the molecules.

In this document, the " 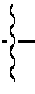 " in the description of groups in the invention is used to denote the position of substitution on the group.

In this document, "liposome" or "lipid nanoparticle (LNP)" refers to a drug delivery system where drugs or other bioactive substances are dissolved or encapsulated within a lipid core, or adsorbed or attached to the surface of nanoparticles, using biocompatible lipid materials as carriers.

In this document, the term "pharmaceutically acceptable excipients" includes any solvents, dispersion media, coating materials, surfactants, antioxidants, preservatives (such as antibacterials and antifungals), isotonic agents, salts, drug stabilizers, binders, mold release agents, dispersing agents, lubricants, sweeteners, flavoring agents, coloring agents, or their combinations, which are known to those skilled in the art (as described in Remington's Pharmaceutical Sciences, 18th Ed. Mack Printing Company, 1990, pp. 1289-1329). This includes their use in therapeutic or pharmaceutical compositions, except where any conventional carrier is incompatible with the active ingredient.

In this document, the term "treatment" refers to the administration to achieve a desired pharmacological and/or physiological effect. The effect can be prophylactic in terms of completely or partly preventing a disease or symptoms thereof, and/or therapeutic in terms of partially or completely curing a disease and/or adverse effects attributable to the disease. The term "treatment" used here covers diseases in mammals, particularly humans, including: (a) preventing the occurrence of a disease or condition in a subject who may be prone but has not yet been diagnosed; (b) inhibiting a disease, such as arresting its development; or (c) relieving a disease, such as alleviating symptoms associated with the disease. The term "treatment" includes administering any drug or compound to an individual to treat, cure, alleviate, ameliorate, mitigate, or inhibit the disease, including but not limited to administering a drug containing the compounds described herein to an individual in need thereof.

This invention introduces a compound and its applications, liposomes, drug carriers, complexes, pharmaceutical compositions, and their pharmaceutical uses. Each of these will be detailed below.

### Compounds

In one aspect of the invention, a compound is proposed, which is the compound represented by formula (I), or a stereoisomer, tautomer, solvate, or pharmaceutically acceptable salt of the compound represented by formula (I):

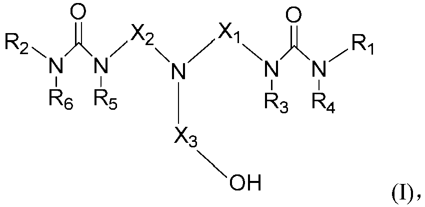

In the invention, the compound is defined where X₁, X₂, and X₃ are each independently selected from optionally substituted C₁-C₁₅ alkyl groups; R₁ and R₂ are each independently selected from optionally substituted C₁-C₄₀ alkyl, optionally substituted C₁-C₄₀ heteroalkyl, optionally substituted C₂-C₄₀ alkenyl, optionally substituted C₂-C₄₀ heteroalkenyl, optionally substituted C₂-C₄₀ alkynyl, or optionally substituted C₂-C₄₀ heteroalkynyl; R₃, R₄, R₅, and R₆ are each independently selected from H, halogen, or optionally substituted C₁-C₃ alkyl. The substituents are independently selected from one or more of halogen, -OH, -SH, -NH₂, -NO₂, cyanide, or C₁-C₃ alkyl.

The compound of this invention exhibits low cytotoxicity and demonstrates good biocompatibility; it has strong delivery capabilities, serving as a delivery carrier for transporting nucleic acid drugs and other substances within an animal body, for example, to the heart, liver, spleen, lungs, and kidneys, especially effective in targeting the spleen to activate the body's immune response and elevate the levels of specific antibodies within the animal.

In some embodiments of the invention, X₁ and X₂ are each independently a C₄-C₁₂ alkyl group. In other embodiments, X₃ is a C₂-C₈ alkyl group. In certain scenarios, X₁ as a C₄ alkyl group, X₂ as a C₄ alkyl group, and X₃ as a C₂ alkyl group do not coexist simultaneously.

In some implementations, R₃, R₄, R₅, and R6 are each independently hydrogen (H) or a halogen.

Furthermore, in some embodiments, R₁ and R₂ each independently have a structure represented by formula (II): 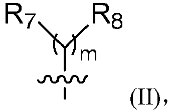 wherein m is an integer ranging from 1 to 10. R7 and R8 are each independently hydrogen (H), optionally substituted C₁-C₂₀ alkyl, optionally substituted C₁-C₂₀ heteroalkyl, optionally substituted C₂-C₂₀ alkenyl, optionally substituted C2-C20 heteroalkenyl, optionally substituted C₂-C₂₀ alkynyl, or optionally substituted C₂-C₂₀ heteroalkynyl. The substituents on these groups are independently selected from one or more of halogens, -OH, -SH, -NH₂, -NO₂, cyanide, or C₁-C₃ alkyl.

In some preferred embodiments, "m" is 2, 3, or 4. For example, when "m" is 3, R₁ and R₂ have the following structure:

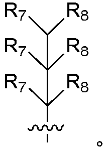

In some embodiments, R₇ and R₈ are each independently hydrogen (H), optionally substituted C₄-C₁₈ alkyl, optionally substituted C₄-C₁₈ heteroalkyl, optionally substituted C₄-C₁₈ alkenyl, or optionally substituted C₄-C₁₈ heteroalkenyl, where the substituent groups are independently selected from one or more of halogens, -OH, -SH, -NH₂, -NO₂, cyanide, or C₁-C₃ alkyl.

In some embodiments, R₁ and R₂ each independently have a structure as represented by formula (III): 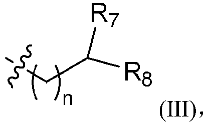 where n is 1 or 2; R₇ and R8 are each independently hydrogen, optionally substituted C₁-C₂₀ alkyl, optionally substituted C₁-C₂₀ heteroalkyl, optionally substituted C₂-C₂₀ alkenyl, optionally substituted C₂-C₂₀ heteroalkenyl, optionally substituted C₂-C₂₀ alkynyl, or optionally substituted C₂-C₂₀ heteroalkynyl, where the substituent groups are independently selected from one or more of halogens, -OH, -SH, -NH₂, -NO₂, cyanide, or C₁-C₃ alkyl.

In some preferred embodiments, R₇ and R8 are each independently optionally substituted C₄-C₁₈ alkyl, optionally substituted C₄-C₁₈ heteroalkyl, optionally substituted C₄-C₁₈ alkenyl, or optionally substituted C₄-C₁₈ heteroalkenyl, where the substituent groups are independently selected from one or more of halogens, -OH, -SH, -NH₂, -NO₂, cyanide, or C₁-C₃ alkyl.

In some embodiments, R₁ and R₂ each independently have at least one of the following structures:

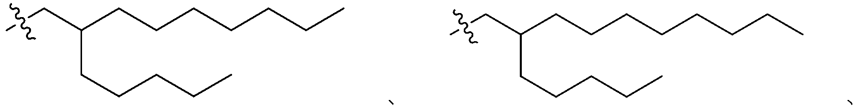

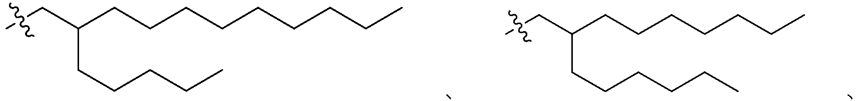

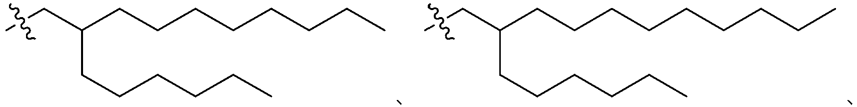

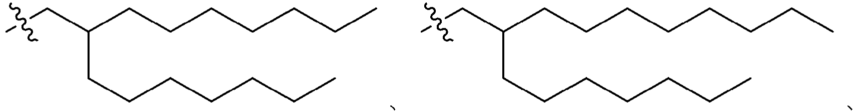

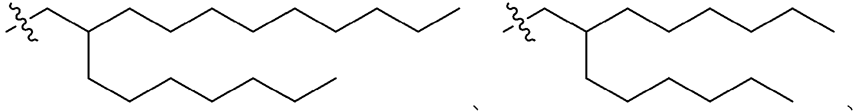

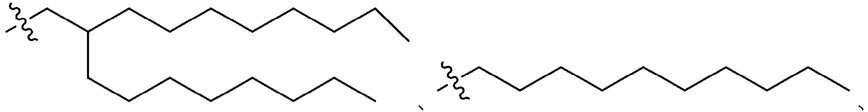

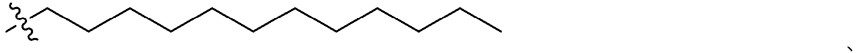

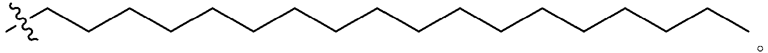

In some embodiments, the compound has at least one of the following structures:

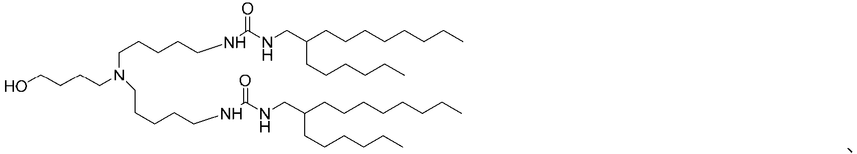

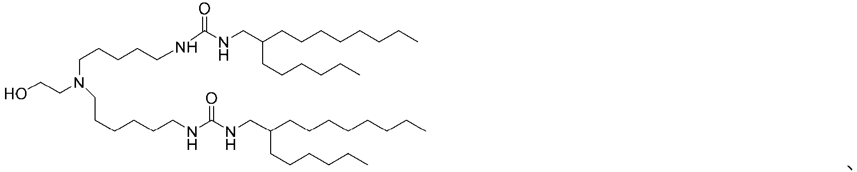

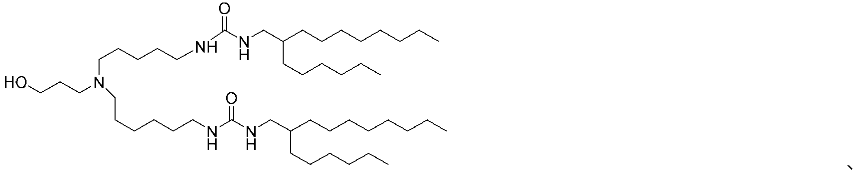

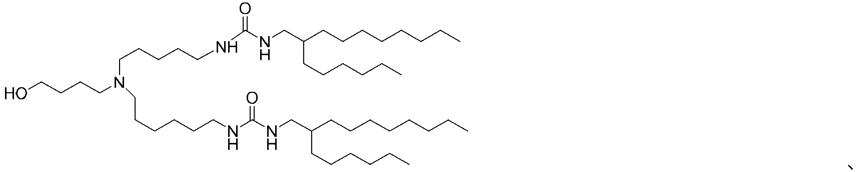

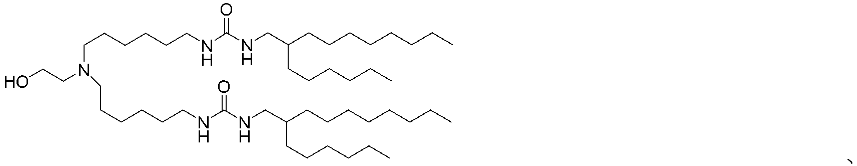

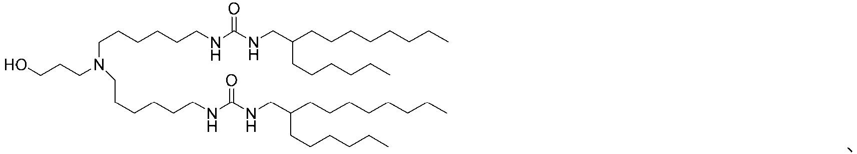

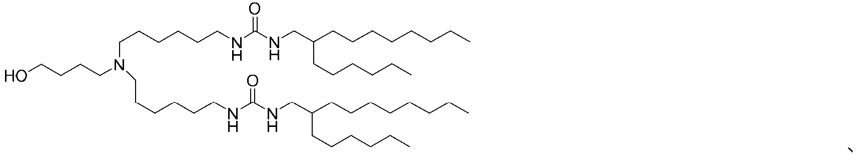

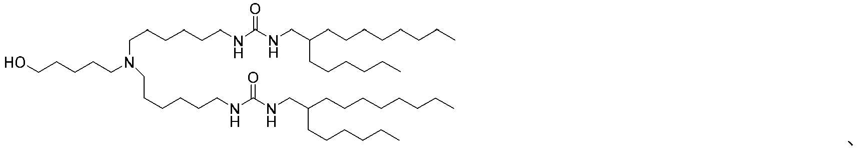

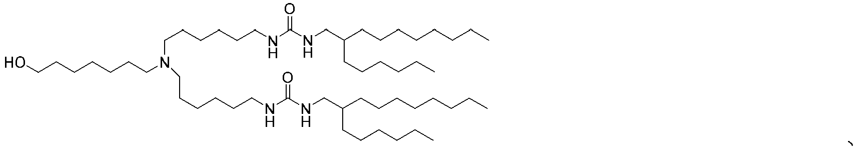

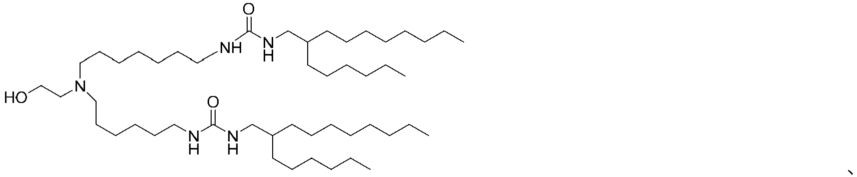

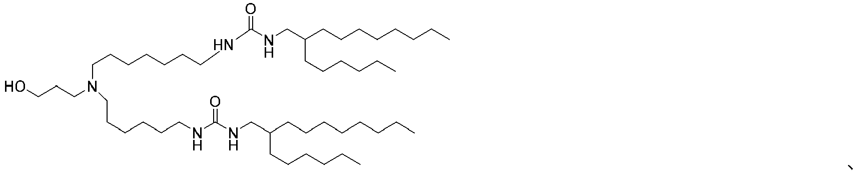

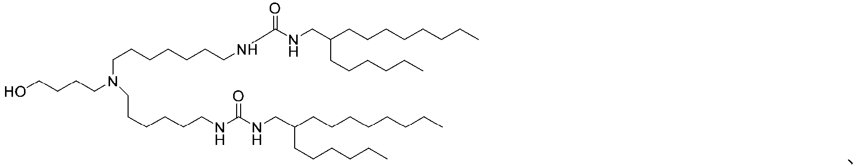

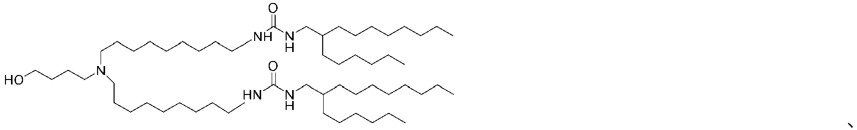

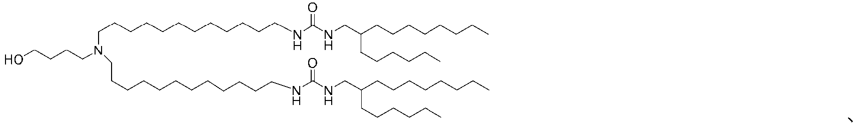

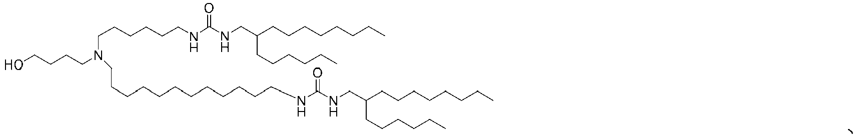

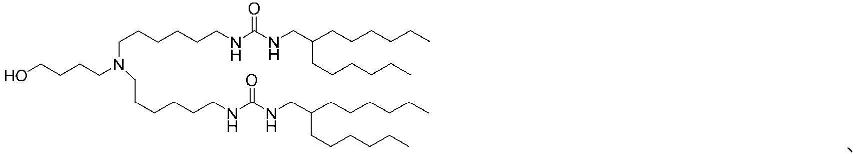

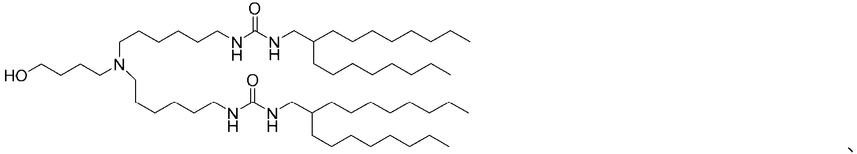

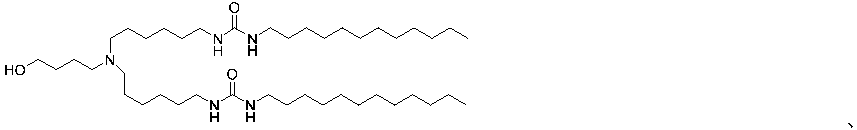

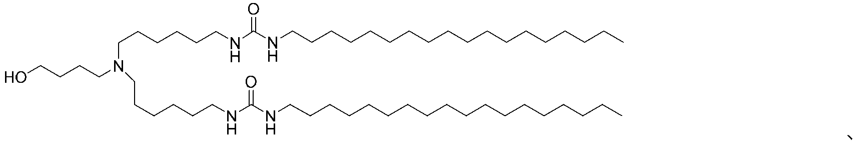

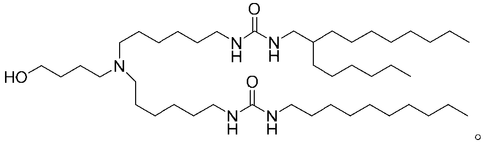

In some embodiments, the compounds of this invention also include other salts of the aforementioned compounds, which are not necessarily pharmaceutically acceptable salts. These other salts can be used as intermediates in the preparation and/or purification of the compounds of this invention or in the separation of enantiomers of the compounds.

The compounds of this invention or their salts may also be obtained in their hydrated forms, or may include other solvents used for their crystallization. The compounds of this invention can inherently or by design form solvates with pharmaceutically acceptable solvents (including water); therefore, this invention intends to encompass both solvated and unsolvated forms.

Any structural formulas provided in this invention represent the compounds in their unmarked (non-isotopically labeled) forms. However, the compounds of this invention also include versions labeled with various isotopes as defined by the invention. Apart from one or more atoms being replaced by atoms having selected atomic masses or mass numbers, isotopically labeled compounds possess the same structures depicted by the general formulas given in this invention. The isotopes that can be introduced into the compounds of this invention include isotopes of hydrogen, carbon, nitrogen, oxygen, phosphorus, sulfur, fluorine, and chlorine, such as ²H (deuterium), ³H (tritium), ¹¹C, ¹³C, ¹⁴C, ¹⁵N, ¹⁸F, ³¹P, ³²P, ³⁶S, ³⁷Cl, or ¹²⁵I.

In another aspect of this invention, a use of the aforementioned compound in the preparation of liposomes, drug carriers, or complexes is proposed. According to embodiments of the invention, the compound possesses ionizable properties, making it suitable for the preparation of liposomes. The liposomes prepared can serve as drug carriers, forming complexes with nucleic acid drugs.

In some embodiments, the compound can be used in the form of polymers to prepare liposomes; it can form covalent bonds with other substances to produce liposomes; or it can undergo chemical reactions with other substances to fabricate liposomes. According to embodiments of this invention, the specific methods of preparing liposomes using the aforementioned compound are not limited. The use of the compound in preparing liposomes, where the liposomes contain all or part of the structure of the aforementioned compound, is considered a utility of this invention.

For administration purposes, the compounds of this invention (typically in the form of liposomes combined with bioactive components) can be administered as bulk chemicals, or they may be formulated into pharmaceutical compositions. The pharmaceutical compositions of this invention contain the compound of structure (I) along with one or more pharmaceutically acceptable carriers, diluents, or excipients. The compound of structure (I) is present in an effective amount to form liposomes and deliver bioactive components, for example, for treating specific diseases or conditions related to the therapeutic application. Those skilled in the art can readily determine appropriate concentrations and dosages.

### Liposomes

In another aspect of the invention, a liposome is proposed. According to an embodiment of the invention, the liposome includes the aforementioned compound. Liposomes in accordance with this invention exhibit low cytotoxicity, good biocompatibility, strong delivery capabilities, and effective immune activation benefits.

In some embodiments, the liposome further includes at least one of the following: steroids, neutral lipids, and PEG-lipids. These components can enhance the stability, longevity, and effectiveness of the liposomes as drug delivery systems, improving their interaction with biological systems and optimizing the delivery of therapeutic agents.

In some embodiments, the neutral lipids included in the liposome are selected from the following: 1,2-distearoyl-sn-glycero-3-phosphocholine (DSPC), 1,2-dipalmitoyl-sn-glycero-3-phosphocholine (DPPC), 1,2-dipalmitoyl-sn-glycero-3-phosphoethanolamine (DPPE), 1,2-dimyristoyl-sn-glycero-3-phosphocholine (DMPC), 1,2-dimyristoyl-sn-glycero-3-phosphoethanolamine (DMPE), 1-palmitoyl-2-oleoyl-sn-glycero-3-phosphocholine (POPC), 1,2-dioleoyl-sn-glycero-3-phosphocholine (DOPC), 2-dioleoyl-sn-glycerol-3-phosphate (1'-rac-glycerol) (DOPG), 1,2-dioleoyl-sn-glycero-3-phosphoethanolamine (DOPE), and sphingomyelin (SM). These neutral lipids are fundamental components for the formation and structural integrity of the liposome, playing crucial roles in its stability, encapsulation efficiency, and interaction with cellular membranes.

In some embodiments, the PEG-lipids included in the formulation are selected from at least one of the following: 2-[(polyethylene glycol)-2000]-N,N-ditetradecylacetamide (ALC-0159), 1,2-dimyristoyl-sn-glycero-methoxypolyethylene glycol (PEG-DMG), 1,2-distearoyl-sn-glycero-3-phosphoethanolamine-N-[amino(polyethylene glycol)] (PEG-DSPE), PEG-disteroylglycerol (PEG-DSG), PEG-dipalmitoyl, PEG-dioleyl, PEG-distearoyl, PEG-diacylglycerolamide (PEG-DAG), PEG-dipalmitoyl phosphatidylethanolamine (PEG-DPPE), PEG-phosphatidylethanolamine (PEG-PE), PEG-succinate diacylglycerol (PEG-S-DAG), PEG-ceramide (PEG-cer), PEG-dialkoxylpropylaminomethylacetate, and PEG-1,2-dimyristoyloxypropyl-3-amine (PEG-c-DMA). These PEG-lipids are used to enhance the stability, circulation time, and biocompatibility of liposomes, effectively reducing their recognition and clearance by the immune system.

In some embodiments, the steroids include at least one selected from cholesterol, coprostanol, ergosterol, ergocalciferol, brassicasterol, sitosterol, and rapeseed sterol, with cholesterol being preferred.

In some embodiments, the molar ratios of the compound, neutral lipid, steroid, and PEG-lipid are (20~80):(5~50):(10~60):(0.01~10).

In some embodiments, the molar ratios of the compound, neutral lipid, steroid, and PEG-lipid are (40~60):(5~10):(30~50):(0.5~5).

In another aspect of the invention, a liposome is proposed. According to an embodiment of the invention, the liposome includes the aforementioned compound, neutral lipid, cholesterol, and PEG-lipid, where the neutral lipid includes at least one of 1,2-dioleoyl-sn-glycero-3-phosphoethanolamine (DOPE) and/or 1,2-dioleoyl-sn-glycero-3-phosphocholine (DOPC), and the PEG-lipid is 1,2-dimyristoyl-sn-glycero-methoxypolyethylene glycol (PEG-DMG). The molar ratios of the compound, neutral lipid, cholesterol, and PEG-lipid are (40~60):(5~10):(30~50):(0.5~5). The liposome as per the embodiments of this invention exhibits low cytotoxicity, good biocompatibility, strong delivery capabilities, and effective immune activation.

### Drug carriers, complex and pharmaceutical compositions

In another aspect of the invention, a drug carrier is proposed. According to embodiments of the invention, the drug carrier includes the aforementioned compound or liposome. The compound is an ionizable lipid, and the drug carrier is an ionizable carrier, which allows the compound or liposome to load drugs and deliver them into cells, facilitating the effectiveness of the carried drugs for treating related diseases.

Another aspect of the invention introduces a complex. According to embodiments of the invention, the complex includes the aforementioned compound, liposome, or drug carrier, along with a bioactive component. As previously stated, the compound is an ionizable lipid, and the drug carrier is an ionizable carrier, which allows for the preparation of a complex containing bioactive components. These complexes can deliver drugs into the body's cells, enhancing the therapeutic effects of the bioactive components for treating diseases.

In some embodiments, the bioactive component includes at least one selected from DNA molecules, RNA molecules, proteins, peptides, and small molecule drugs.

In some implementations, the proteins or peptides are not specifically limited and can be peptide chains, the proteins themselves, derivatives thereof, or complexes with other substances, such as Cas9 protein or its partial structural domain peptides, radio-labelled proteins, antibodies, etc.

In some embodiments, the bioactive component is a nucleic acid. For example, the bioactive component can be antisense RNA and messenger RNA.

In some embodiments, the bioactive component contains DNA, RNA, nucleic acid-protein complexes, nucleic acid-lipid complexes, nucleic acid-radionuclide complexes, etc., without specific restrictions on the type of nucleic acid. Specific examples of such nucleic acids can include siRNA, mRNA, tRNA, rRNA, cDNA, miRNA (microRNA), ribozymes, antisense oligonucleotides, plasmid DNA, peptide nucleic acids, triplex forming oligonucleotides (TFO), genes, etc. The nucleic acids applicable in the drug carriers of this invention can be derived from humans, animals, plants, bacteria, viruses, etc., and can also be chemically synthesized. Furthermore, the nucleic acids can be single-stranded, double-stranded, or triple-stranded, and there are no specific restrictions on their molecular weight. Additionally, in this invention, nucleic acids can be chemically, enzymatically, or peptide-modified. In the invention, nucleic acids can be used alone or in a suitable combination of two or more. In some embodiments, the nucleic acid transport carrier compositions of this invention preferably transport mRNA or its analogs.

In some embodiments, the bioactive component is negatively charged or hydrophobic. According to embodiments of the invention, the drug carrier contains ionizable lipids that are positively charged at specific pH levels, enabling binding with negatively charged bioactive components.

Another aspect of the invention introduces a pharmaceutical composition. According to embodiments of the invention, the pharmaceutical composition includes the aforementioned compound, liposome, drug carrier, or complex. As previously described, the compound, liposome, drug carrier, and complex possess advantages such as low cytotoxicity, good biocompatibility, strong delivery capability, and effective immune activation, delivering bioactive components within the body to exert therapeutic effects for disease treatment.

In some embodiments, the pharmaceutical composition includes pharmaceutically acceptable excipients.

The administration of the pharmaceutical compositions of this invention can be performed by any acceptable method. The compositions can be prepared in solid, semi-solid, liquid, or gaseous forms, such as tablets, capsules, powders, granules, ointments, solutions, suspensions, suppositories, injections, inhalants, gels, microspheres, and aerosols. The methods for preparing these dosage forms are known or obvious to those skilled in the art. Typical routes of administration for these pharmaceutical compositions include, but are not limited to, oral, topical, transdermal, inhalational, parenteral, sublingual, buccal, rectal, vaginal, and nasal routes. The term "parenteral" includes subcutaneous, intravenous, intramuscular, intradermal, sternum intravenous injection, or infusion techniques. The pharmaceutical compositions are prepared to allow the bioactive components contained within them to be bioavailable after administration to a patient. The compositions to be administered to a subject or patient are in the form of one or more dosage units, where, for example, a tablet can be a single dosage unit, while a container of the invention's aerosol form may hold multiple dosage units.

### Applications

In another aspect of the invention, the proposed uses of the aforementioned compound, liposome, drug carrier, complex, or pharmaceutical composition are in the preparation of medications. These medications are intended for targeting specific organs such as the heart, liver, spleen, lungs, and kidneys. The compound, liposome, drug carrier, and complex are noted for their low cytotoxicity, good biocompatibility, strong delivery capabilities, and effective immune activation, making them effective for delivering bioactive components to these organs to treat diseases.

Specifically, some embodiments focus on targeting the spleen. The term "targeting" implies delivering the bioactive component loaded in the liposome to a predetermined target, resulting in a significant presence of the bioactive component in that target organ, which in this case is non-specifically the spleen.

Furthermore, the invention outlines the use of the compound, liposome, drug carrier, complex, or pharmaceutical composition in the treatment or prevention of diseases, leveraging their beneficial properties to deliver bioactive components effectively within the body. This application is particularly crucial for diseases including but not limited to tumors and tumor-related diseases, and diseases caused by viruses.

Some implementations detail the types of tumors that may be targeted, including melanoma, brain tumors, esophageal cancer, stomach cancer, liver cancer, pancreatic cancer, colorectal cancer, lung cancer, kidney cancer, breast cancer, cervical cancer, ovarian cancer, prostate cancer, skin cancer, neuroblastoma, glioma, sarcomas, bone cancer, uterine cancer, endometrial cancer, head and neck tumors, multiple myeloma, B-cell lymphoma, polycythemia vera, leukemia, thyroid tumors, bladder cancer, or gallbladder cancer.

In some instances, the virus-related diseases could include those related to coronaviruses.

The medications developed using these compounds can deliver bioactive components to the spleen and other organs. The inventors have discovered that compared to traditional liposomes or drug carriers, the use of the compounds, liposomes, or drug carriers of this invention can increase the concentration or expression of bioactive components in the spleen, thus offering therapeutic benefits for spleen-related diseases.

### Methodology

In yet another aspect, the invention proposes a method for treating or preventing diseases. This method involves administering an acceptable pharmaceutical amount of the aforementioned complex or pharmaceutical composition to a subject. The effective amount of the compound or pharmaceutical composition can vary based on the mode of administration and the severity of the disease being treated. Preference for the effective amount can be determined by those skilled in the art (e.g., through clinical trials) based on various factors such as the pharmacokinetic parameters of the bioactive component including bioavailability, metabolism, half-life, the severity of the disease, patient's weight, immune status, and the route of administration. For urgent treatment conditions, multiple separated doses may be given daily, or doses may be adjusted proportionally.

The compound or pharmaceutical composition can be incorporated into a medication suitable for parenteral administration (e.g., intravenous, subcutaneous, intraperitoneal, intramuscular). These medications can be prepared in various forms such as liquid, semi-solid, and solid dosage forms including but not limited to liquid solutions (e.g., injectable solutions and infusions) or lyophilized powders. Typically, the medication is in the form of an injectable solution. The aforementioned complex or pharmaceutical composition can be administered via intravenous infusion or injection, intramuscular injection, or subcutaneous injection.

The route of administration in the examples of this invention may include intramuscular or intravenous injections.

Further explanation of this invention's schemes will be detailed in the embodiments. Those skilled in the art will understand that the following examples are for illustrative purposes only and should not be seen as limiting the scope of the invention. Where specific techniques or conditions are not stated in the embodiments, they are carried out according to techniques or conditions described in the literature in the field, or according to the manufacturer's instructions of the products used. All reagents or instruments not specified by the manufacturer are conventional products that can be obtained commercially.

### Example 1: Preparation of Compound 7

Synthesis Route and Characterization of Compound 7:

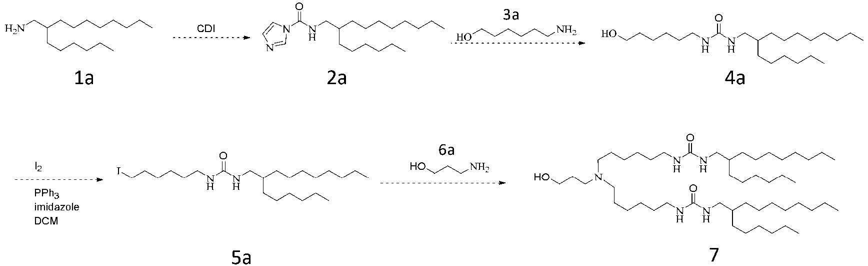

Synthesis of Compound 2a: at room temperature, compound 1a (2.0 g, 8.3 mmol) and CDI (2.0 g, 12.3 mmol, i.e., N,N'-carbonyldiimidazole) are added to a DCM (85.0 mL, i.e., dichloromethane) solution. The reaction mixture is stirred overnight at room temperature. TLC indicates complete consumption of 1a. The reaction mixture is then added to water and extracted with DCM. The organic phase is washed with brine, dried over Na₂SO₄, and concentrated. The crude product of compound 2a is used directly in the next step without purification.

Synthesis of Compound 4a: at room temperature, compound 2a (2.5 g, 7.5 mmol) and compound 3a (1.8 g, 15 mmol) are added to a DCM (75.0 mL) solution. The reaction mixture is stirred overnight at room temperature. TLC indicates that the reaction of compound 2a is complete. The reaction mixture is then added to water and extracted with DCM. The organic phase is washed with brine, dried over Na₂SO₄, and then concentrated. Compound 4a is purified by column chromatography (PE: EA = 7:1 to 1:1).

Synthesis of Compound 5a: at room temperature, add compound 4a (2.1 g, 5.5 mmol), I₂ (2.2 g, 8.7 mmol), PPh₃ (2.3 g, 8.8 mmol, i.e., triphenylphosphine), and imidazole (0.6 g, 6.6 mmol) to a DCM (20.0 mL) solution. Stir the reaction mixture at 60°C for 3 hours. LCMS indicates complete reaction of compound 4a. Add the reaction mixture to water and extract with ethyl acetate (EA). Wash the organic phase with brine, dry over Na₂SO₄, and then concentrate. Purify compound 5a by column chromatography (PE: EA = 9:1 to 7:1).

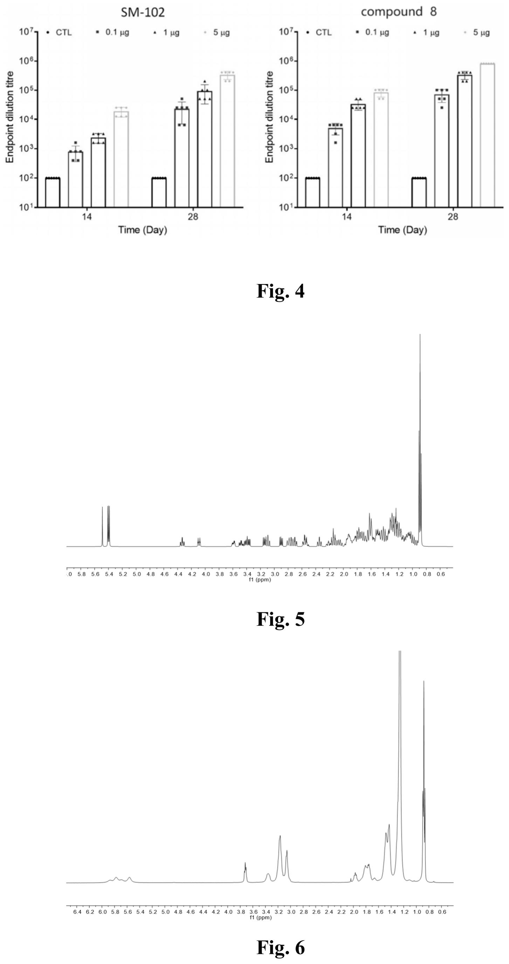

Synthesis of Compound 7: at room temperature, dissolve compound 5a (3.3 g, 6.6 mmol), compound 6a, and K₂CO₃ (1.5 g, 9.3 mmol) in a DCM (10.0 mL) solution. Stir the reaction mixture at 40°C for 3 hours. TLC indicates that compound 5a is completely consumed. Add the reaction mixture to water and extract with ethyl acetate (EA). Wash the organic phase with brine, dry over Na₂SO₄, and then concentrate. Purify compound 7 by column chromatography (DCM : MeOH = 150:0 to 20:1). The H1-NMR spectrum of compound 7 is shown in Figure 5.

### Example 2: Preparation of Compounds 8 to 21

### Synthesis Route and Characterization of Compound 8:

According to the method described in Example 1, compound 2a, compound 4a and compound 5a were synthesized in sequence.

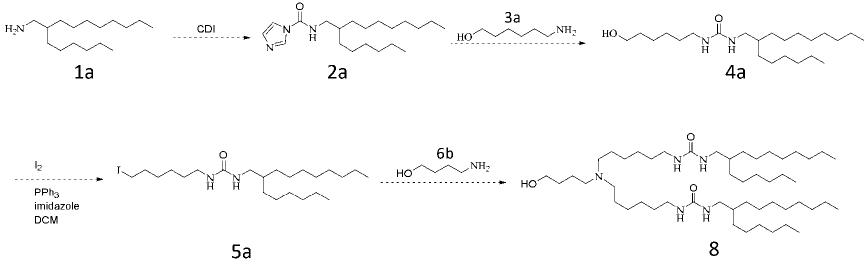

Compound 8 Synthesis: at room temperature, compound 5a (3.3 g, 6.6 mmol), compound 6b, and K₂CO₃ (1.5 g, 9.3 mmol) are dissolved in a DCM (10.0 mL) solution. The reaction mixture is stirred at 40°C for 3 hours. TLC indicates complete consumption of compound 5a. The reaction mixture is then poured into water and extracted with EA. The organic phase is washed with brine, dried over Na₂SO₄, and concentrated. Compound 8 is purified by column chromatography (DCM: MeOH = 150:0-20:1). The H1-NMR spectrum is depicted in Figure 6.

### Synthesis Route and Characterization of Compound 9:

According to the method described in Example 1, compound 2a, compound 4a and compound 5a were synthesized in sequence.

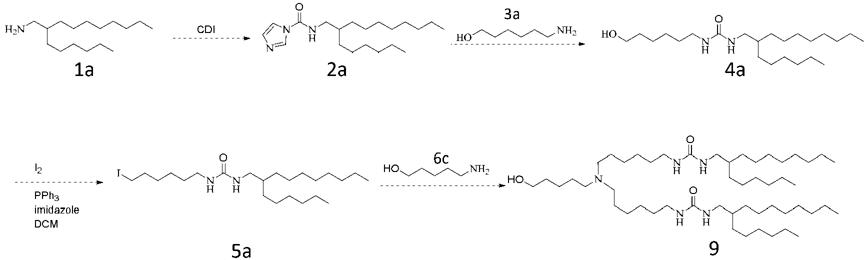

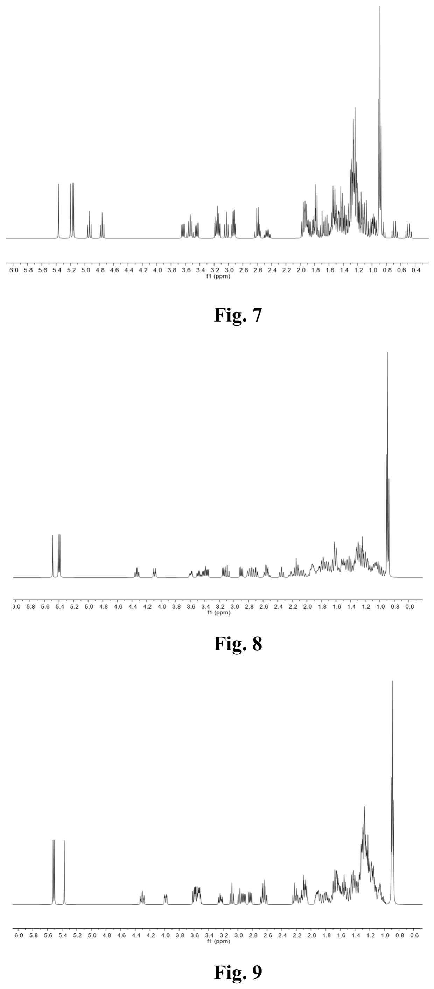

Compound 9 Synthesis: at room temperature, compound 5a (3.3 g, 6.6 mmol), compound 6c, and K₂CO₃ (1.5 g, 9.3 mmol) are dissolved in a DCM (10.0 mL) solution. The reaction mixture is stirred at 40°C for 3 hours. TLC indicates complete consumption of compound 5a. The reaction mixture is then poured into water and extracted with EA. The organic phase is washed with brine, dried over Na₂SO₄, and concentrated. Compound 9 is purified by column chromatography (DCM: MeOH = 150:0-20:1). The H1-NMR spectrum is depicted in Figure 7.

### Synthesis Route and Characterization of Compound 10:

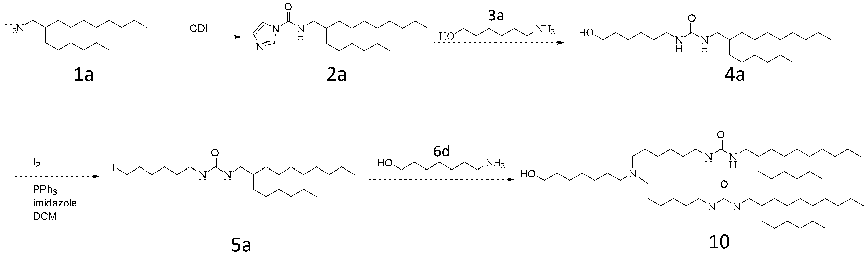

According to the method described in Example 1, compound 2a, compound 4a and compound 5a were synthesized in sequence.

Compound 10 Synthesis: at room temperature, compound 5a (3.3 g, 6.6 mmol), compound 6d, and K₂CO₃ (1.5 g, 9.3 mmol) are dissolved in a DCM (10.0 mL) solution. The reaction mixture is stirred at 40°C for 3 hours. TLC indicates complete consumption of compound 5a. The reaction mixture is then poured into water and extracted with EA. The organic phase is washed with brine, dried over Na₂SO₄, and concentrated. Compound 10 is purified by column chromatography (DCM: MeOH = 150:0-20:1). The H1-NMR spectrum is depicted in Figure 8.

### Synthesis Route and Characterization of Compound 11:

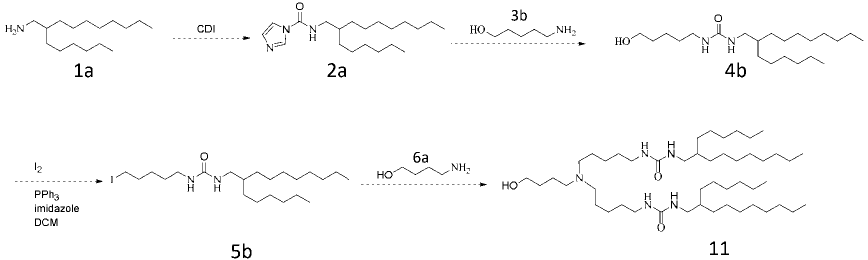

Compound 2a was synthesized according to the method described in Example 1.

Compound 4b Synthesis: at room temperature, compound 2a (2.5 g, 7.5 mmol) and compound 3b (1.5 g, 15 mmol) are added to a DCM (75.0 mL) solution. The reaction mixture is stirred overnight at room temperature. TLC shows complete reaction of compound 2a. The reaction mixture is poured into water and extracted with DCM. The organic phase is washed with brine, dried over Na₂SO₄, and then concentrated. Compound 4b is purified by column chromatography (PE: EA = 7:1-1:1).

Compound 5b Synthesis: at room temperature, compound 4b (2.0 g, 5.5 mmol), I₂ (2.2 g, 8.7 mmol), PPh₃ (2.3 g, 8.8 mmol), and imidazole (0.6 g, 6.6 mmol) are added to a DCM (20.0 mL) solution. The reaction mixture is stirred at 60°C for 3 hours. LCMS shows complete reaction of compound 4b. The reaction mixture is poured into water and extracted with EA. The organic phase is washed with brine, dried over Na₂SO₄, and then concentrated. Compound 5b is purified by column chromatography (PE: EA = 9:1-7:1).

Compound 11 Synthesis: at room temperature, compound 5b (3.2 g, 6.6 mmol), 6a, and K₂CO₃ (1.5 g, 9.3 mmol) are dissolved in DCM (10.0 mL). The reaction mixture is stirred at 40°C for 3 hours. TLC shows complete consumption of compound 5b. The reaction mixture is poured into water and extracted with EA. The organic phase is washed with brine, dried over Na₂SO₄, and then concentrated. Compound 11 is purified by column chromatography (DCM: MeOH = 150:0-20:1).

### Synthesis Route and Characterization of Compound 12::

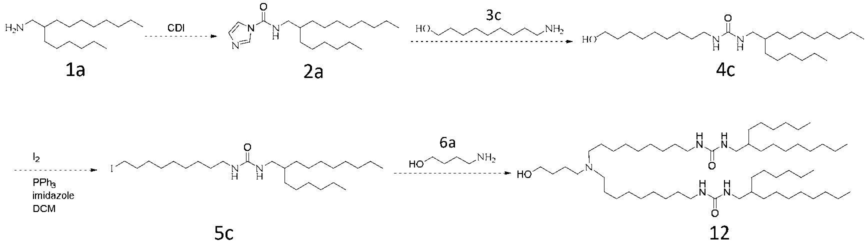

Compound 2a was synthesized according to the method described in Example 1.

Compound 4c Synthesis: at room temperature, compound 2a (2.5 g, 7.5 mmol) and compound 3c (2.4 g, 15 mmol) are added to a DCM (75.0 mL) solution. The reaction mixture is stirred overnight at room temperature. LCMS shows complete reaction of compound 3c. The reaction mixture is poured into water and extracted with DCM. The organic phase is washed with brine, dried over Na₂SO₄, and then concentrated. Compound 4c is purified by column chromatography (PE: EA= 7:1-1:1).

Compound 5c Synthesis: at room temperature, compound 4c (2.3 g, 5.5 mmol), I₂ (2.2 g, 8.7 mmol), PPh₃ (2.3 g, 8.8 mmol), and imidazole (0.6 g, 6.6 mmol) are added to a DCM (20.0 mL) solution. The reaction mixture is stirred at 60°C for 3 hours. LCMS shows complete reaction of compound 4c. The reaction mixture is poured into water and extracted with EA. The organic phase is washed with brine, dried over Na₂SO₄, and then concentrated. Compound 5c is purified by column chromatography (PE: EA = 9:1-7:1).

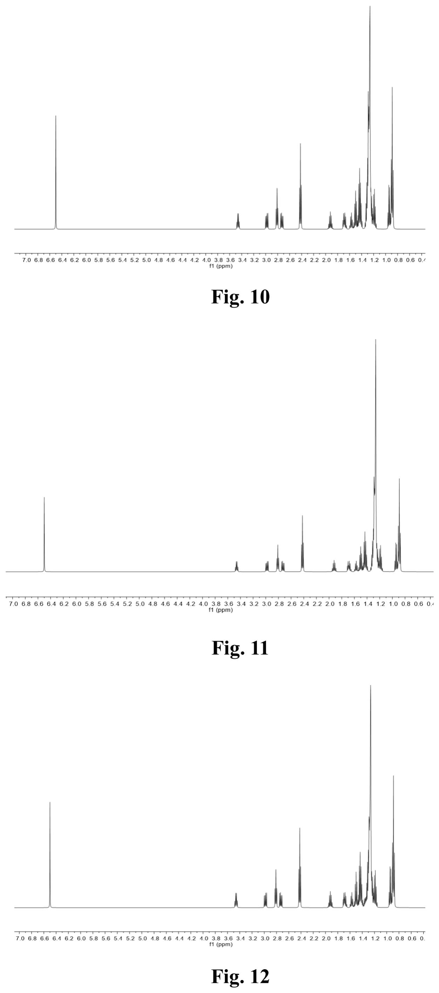

Compound 12 Synthesis: at room temperature, compound 5c (3.5 g, 6.6 mmol), 6a, and K₂CO₃ (1.5 g, 9.3 mmol) are dissolved in DCM (10.0 mL). The reaction mixture is stirred at 40°C for 3 hours. TLC shows complete consumption of compound 5c. The reaction mixture is poured into water and extracted with EA. The organic phase is washed with brine, dried over Na₂SO₄, and then concentrated. Compound 12 is purified by column chromatography (DCM: MeOH = 150:0-20:1). The H1-NMR spectrum is shown in Figure 10.

### Synthesis Route and Characterization of Compound 13::

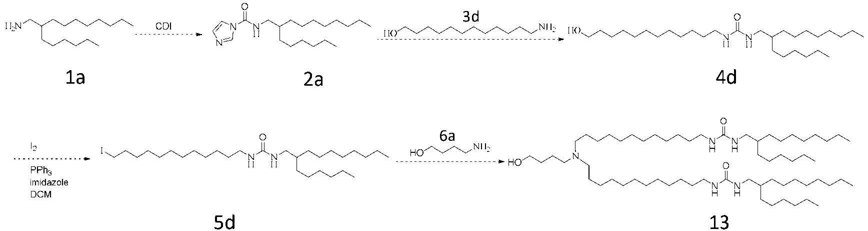

Compound 2a was synthesized according to the method described in Example 1.

Compound 4d Synthesis: at room temperature, compound 2a (2.5 g, 7.5 mmol) and compound 3d (3.0 g, 15 mmol) are added to a DCM (75.0 mL) solution. The reaction mixture is stirred overnight at room temperature. LCMS shows complete reaction of compound 3d. The reaction mixture is poured into water and extracted with DCM. The organic phase is washed with brine, dried over Na₂SO₄, and then concentrated. Compound 4d is purified by column chromatography (PE: EA = 7:1-1:1).

Compound 5d Synthesis: at room temperature, compound 4d (2.6 g, 5.5 mmol), I₂ (2.2 g, 8.7 mmol), PPh₃ (2.3 g, 8.8 mmol), and imidazole (0.6 g, 6.6 mmol) are added to a DCM (20.0 mL) solution. The reaction mixture is stirred at 60°C for 3 hours. LCMS shows complete reaction of compound 4d. The reaction mixture is poured into water and extracted with EA. The organic phase is washed with brine, dried over Na₂SO₄, and then concentrated. Compound 5d is purified by column chromatography (PE: EA = 9:1-7:1).

Compound 13 Synthesis: at room temperature, compound 5d (3.8 g, 6.6 mmol), 6a, and K₂CO₃ (1.5 g, 9.3 mmol) are dissolved in DCM (10.0 mL). The reaction mixture is stirred at 40°C for 3 hours. TLC shows complete consumption of compound 5d. The reaction mixture is poured into water and extracted with EA. The organic phase is washed with brine, dried over Na₂SO₄, and then concentrated. Compound 13 is purified by column chromatography (DCM: MeOH = 150:0-20:1). The H1-NMR spectrum is shown in Figure 11.

### Synthesis Route and Characterization of Compound 15:

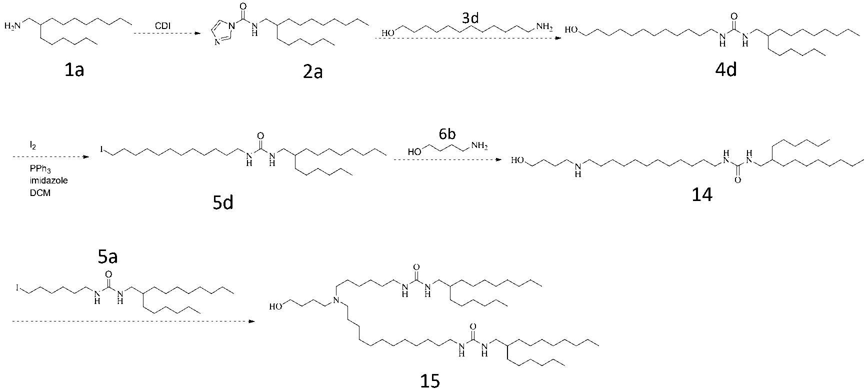

Compound 5d was synthesized according to the method described for compound 13.

Compound 14 Synthesis: at room temperature, compound 5d (3.6 g, 6.6 mmol), compound 6b (0.6 g, 6.6 mmol), and K₂CO₃ (1.5 g, 9.3 mmol) are dissolved in DCM (10.0 mL). The reaction mixture is stirred at 40°C for 3 hours. TLC shows complete consumption of compound 5d. The reaction mixture is poured into water and extracted with EA. The organic phase is washed with brine, dried over Na₂SO₄, and then concentrated. Compound 14 is purified by column chromatography (DCM: MeOH = 150:0-20:1).

Synthesis of Compound 5a According to Compound 13.

Compound 15 Synthesis: at room temperature, compound 14 (3.6 g, 6.6 mmol), compound 5a (4.0 g, 8.0 mmol), and K2CO3 (1.5 g, 9.3 mmol) are dissolved in DCM (10.0 mL). The reaction mixture is stirred at 40°C for 3 hours. TLC shows complete consumption of compound 14. The reaction mixture is poured into water and extracted with EA. The organic phase is washed with brine, dried over Na₂SO₄, and then concentrated. Compound 15 is purified by column chromatography (DCM: MeOH = 150:0-20:1). The H1-NMR spectrum is shown in Figure 12.

### Synthesis Route and Characterization of Compound 16:

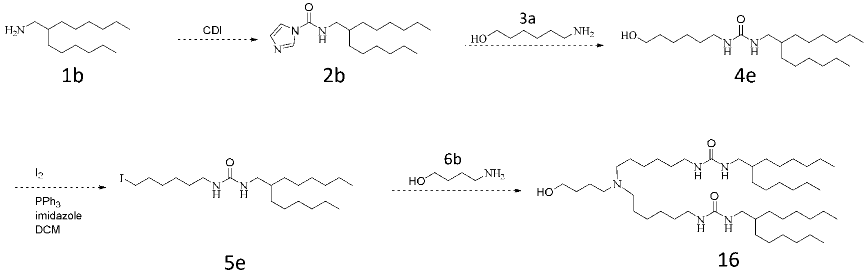

Compound 2b Synthesis: at room temperature, compound 1b (1.8 g, 8.3 mmol) and CDI (2.0 g, 12.3 mmol) are added to a DCM (85.0 mL) solution. The reaction mixture is stirred overnight at room temperature. TLC shows complete consumption of 1b. The reaction mixture is poured into water and extracted with DCM. The organic phase is washed with brine, dried over Na₂SO₄, and then concentrated. The crude product of compound 2b is used directly in the next step without purification.

Compound 4e Synthesis: at room temperature, compound 2b (2.3 g, 7.5 mmol) and compound 3a (1.8 g, 15 mmol) are added to a DCM (75.0 mL) solution. The reaction mixture is stirred overnight at room temperature. TLC shows complete reaction of compound 2b. The reaction mixture is poured into water and extracted with DCM. The organic phase is washed with brine, dried over Na₂SO₄, and then concentrated. Compound 4e is purified by column chromatography (PE: EA= 7:1-1:1).

Compound 5e Synthesis: at room temperature, compound 4e (2.0 g, 5.5 mmol), I₂ (2.2 g, 8.7 mmol), PPh₃ (2.3 g, 8.8 mmol), and imidazole (0.6 g, 6.6 mmol) are added to a DCM (20.0 mL) solution. The reaction mixture is stirred at 60°C for 3 hours. LCMS shows complete reaction of compound 4e. The reaction mixture is poured into water and extracted with EA. The organic phase is washed with brine, dried over Na₂SO₄, and then concentrated. Compound 5e is purified by column chromatography (PE: EA = 9:1-7:1).

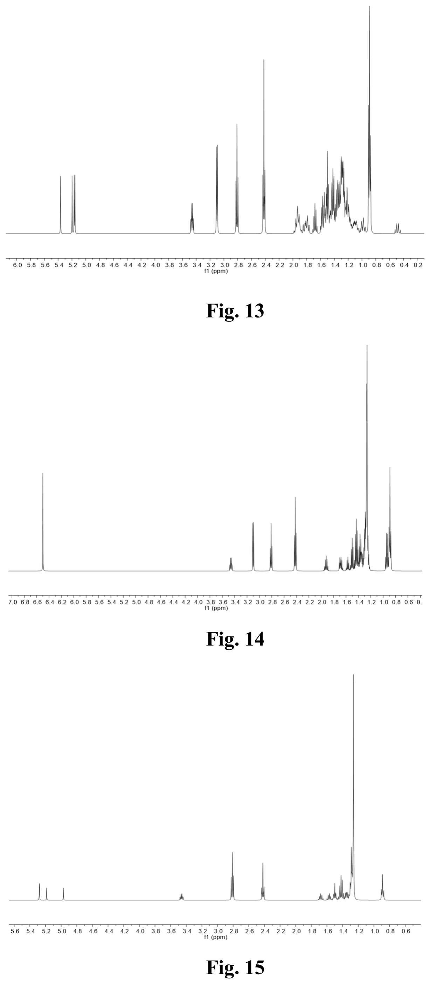

Compound 16 Synthesis: at room temperature, compound 5e (3.1 g, 6.6 mmol), 6a, and K₂CO₃ (1.5 g, 9.3 mmol) are dissolved in DCM (10.0 mL). The reaction mixture is stirred at 40°C for 3 hours. TLC shows complete consumption of compound 5e. The reaction mixture is poured into water and extracted with EA. The organic phase is washed with brine, dried over Na₂SO₄, and then concentrated. Compound 16 is purified by column chromatography (DCM: MeOH = 150:0-20:1). The H1-NMR spectrum is shown in Figure 13.

### Synthesis Route and Characterization of Compound 17:

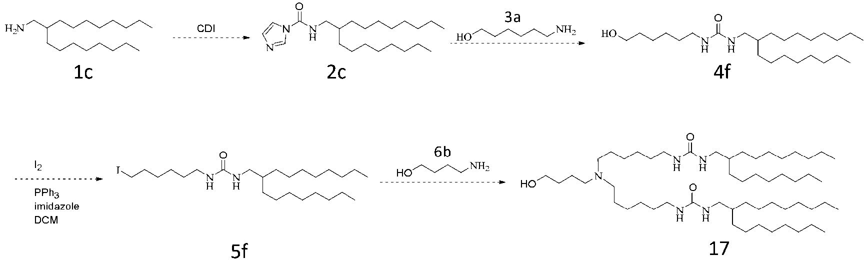

Compound 2c Synthesis: at room temperature, compound 1c (2.2 g, 8.3 mmol) and CDI (2.0 g, 12.3 mmol) are added to a DCM (85.0 mL) solution. The reaction mixture is stirred overnight at room temperature. TLC shows complete consumption of 1b. The reaction mixture is poured into water and extracted with DCM. The organic phase is washed with brine, dried over Na₂SO₄, and then concentrated. The crude product of compound 2c is used directly in the next step without purification.

Compound 4f Synthesis: at room temperature, compound 2c (2.7 g, 7.5 mmol) and compound 3a (1.8 g, 15 mmol) are added to a DCM (75.0 mL) solution. The reaction mixture is stirred overnight at room temperature. TLC shows complete reaction of compound 2c. The reaction mixture is poured into water and extracted with DCM. The organic phase is washed with brine, dried over Na₂SO₄, and then concentrated. Compound 4f is purified by column chromatography (PE: EA= 7:1-1:1).

Compound 5f Synthesis: at room temperature, compound 4f (2.3 g, 5.5 mmol), I₂ (2.2 g, 8.7 mmol), PPh₃ (2.3 g, 8.8 mmol), and imidazole (0.6 g, 6.6 mmol) are added to a DCM (20.0 mL) solution. The reaction mixture is stirred at 60°C for 3 hours. LCMS shows complete reaction of compound 4f. The reaction mixture is poured into water and extracted with EA. The organic phase is washed with brine, dried over Na₂SO₄, and then concentrated. Compound 5f is purified by column chromatography (PE: EA = 9:1-7:1).

Compound 17 Synthesis: at room temperature, compound 5f (3.4 g, 6.6 mmol), 6a, and K₂CO₃ (1.5 g, 9.3 mmol) are dissolved in DCM (10.0 mL). The reaction mixture is stirred at 40°C for 3 hours. TLC shows complete consumption of compound 5f. The reaction mixture is poured into water and extracted with EA. The organic phase is washed with brine, dried over Na₂SO₄, and then concentrated. Compound 17 is purified by column chromatography (DCM: MeOH = 150:0-20:1). The H1-NMR spectrum is shown in Figure 14.

### Synthesis Route and Characterization of Compound 18:

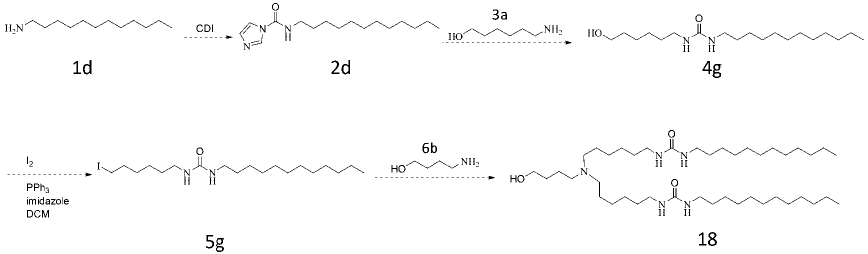

Compound 2d Synthesis: at room temperature, compound 1d (2.2 g, 8.3 mmol) and CDI (2.0 g, 12.3 mmol) are added to a DCM (85.0 mL) solution. The reaction mixture is stirred overnight at room temperature. TLC shows complete consumption of 1b. The reaction mixture is poured into water and extracted with DCM. The organic phase is washed with brine, dried over Na₂SO₄, and then concentrated. The crude product of compound 2d is used directly in the next step without purification.

Compound 4g Synthesis: at room temperature, compound 2d (2.1 g, 7.5 mmol) and compound 3a (1.8 g, 15 mmol) are added to a DCM (75.0 mL) solution. The reaction mixture is stirred overnight at room temperature. TLC shows complete reaction of compound 2d. The reaction mixture is poured into water and extracted with DCM. The organic phase is washed with brine, dried over Na₂SO₄, and then concentrated. Compound 4g is purified by column chromatography (PE: EA = 7:1-1:1).

Compound 5g Synthesis: at room temperature, compound 4g (1.8 g, 5.5 mmol), I₂ (2.2 g, 8.7 mmol), PPh₃ (2.3 g, 8.8 mmol), and imidazole (0.6 g, 6.6 mmol) are added to a DCM (20.0 mL) solution. The reaction mixture is stirred at 60°C for 3 hours. LCMS shows complete reaction of compound 4g. The reaction mixture is poured into water and extracted with EA. The organic phase is washed with brine, dried over Na₂SO₄, and then concentrated. Compound 5g is purified by column chromatography (PE: EA = 9:1-7:1).

Compound 18 Synthesis: at room temperature, compound 5g (2.9 g, 6.6 mmol), 6a, and K₂CO₃ (1.5 g, 9.3 mmol) are dissolved in DCM (10.0 mL). The reaction mixture is stirred at 40°C for 3 hours. TLC shows complete consumption of compound 5g. The reaction mixture is poured into water and extracted with EA. The organic phase is washed with brine, dried over Na₂SO₄, and then concentrated. Compound 18 is purified by column chromatography (DCM: MeOH = 150:0-20:1). The H1-NMR spectrum is shown in Figure 15.

### Synthesis Route and Characterization of Compound 19:

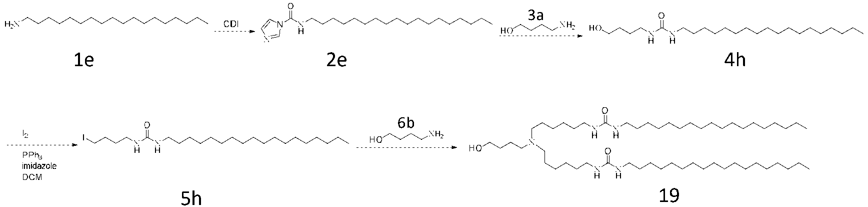

Synthesis of Compound 2e: at room temperature, compound 1e (2.2 g, 8.3 mmol) and CDI (2.0 g, 12.3 mmol) were added to a solution of DCM (85.0 mL). The reaction mixture was stirred overnight at room temperature. TLC analysis showed complete consumption of 1e. The reaction mixture was then poured into water and extracted with DCM. The organic phase was washed with brine, dried over Na₂SO₄, and concentrated. The crude product of compound 2e was used directly in the next step without purification.

Synthesis of Compound 4h: at room temperature, compound 2e (2.7 g, 7.5 mmol) and compound 3a (1.8 g, 15 mmol) were added to a solution of DCM (75.0 mL). The reaction mixture was stirred overnight at room temperature. TLC analysis indicated complete reaction of compound 2e. The reaction mixture was poured into water and extracted with DCM. The organic phase was washed with brine, dried over Na₂SO₄, and concentrated. Compound 4h was purified by column chromatography (PE: EA=7:1-1:1).

Synthesis of Compound 5f: at room temperature, compound 4h (2.1 g, 5.5 mmol), I₂ (2.2 g, 8.7 mmol), PPh₃ (2.3 g, 8.8 mmol), and imidazole (0.6 g, 6.6 mmol) were added to a solution of DCM (20.0 mL). The reaction mixture was stirred at 60°C for 3 hours. LCMS analysis confirmed complete reaction of compound 4h. The reaction mixture was poured into water and extracted with EA. The organic phase was washed with brine, dried over Na₂SO₄, and concentrated. Compound 5f was purified by column chromatography (PE: EA=9:1-7:1).

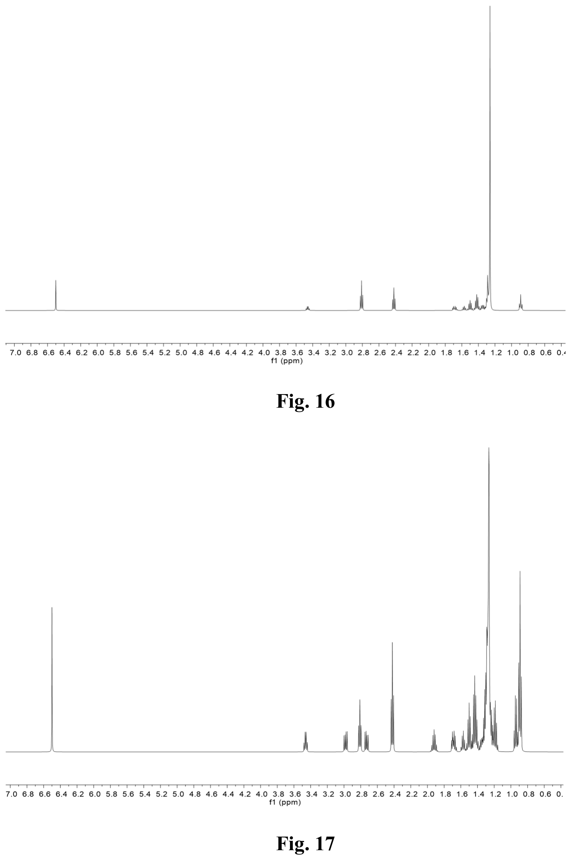

Synthesis of Compound 16: at room temperature, compound 5h (3.3 g, 6.6 mmol), 6b, and K₂CO₃ (1.5 g, 9.3 mmol) were dissolved in DCM (10.0 mL). The reaction mixture was stirred at 40°C for 3 hours. TLC analysis indicated complete consumption of compound 5g. The reaction mixture was poured into water and extracted with EA. The organic phase was washed with brine, dried over Na₂SO₄, and concentrated. Compound 19 was purified by column chromatography (DCM: MeOH=150:0-20:1). The H1-NMR spectrum is shown in Figure 16.

### Synthesis Route and Characterization of Compound 20:

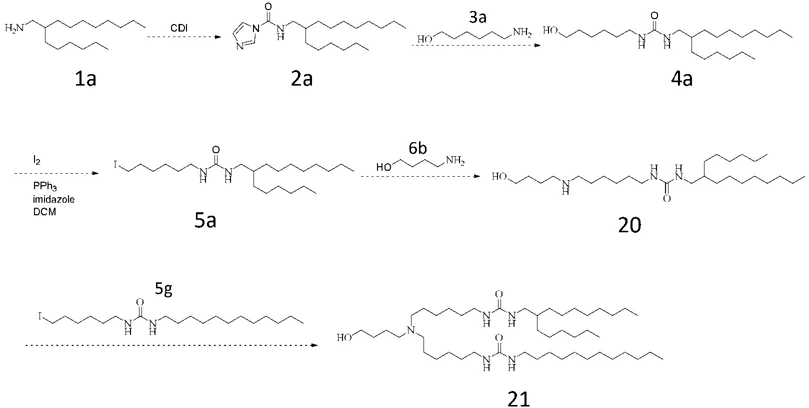

Compound 5a was synthesized according to the method described in Example 1.

Synthesis of Compound 20: at room temperature, compound 5a (3.3 g, 6.6 mmol), compound 6b (0.6 g, 6.6 mmol), and K₂CO₃ (1.5 g, 9.3 mmol) were dissolved in DCM (10.0 mL). The reaction mixture was stirred at 40°C for 3 hours. TLC analysis showed complete consumption of compound 5a. The reaction mixture was then poured into water and extracted with EA. The organic phase was washed with brine, dried over Na₂SO₄, and concentrated. Compound 20 was purified by column chromatography (DCM: MeOH=150:0-20:1).

Synthesis of Compound 21: Following the procedure described for compound 18, compound 5g was synthesized.

Synthesis of Compound 21: at room temperature, compound 20 (3.0 g, 6.6 mmol), compound 5g (3.5 g, 8.0 mmol), and K₂CO₃ (1.5 g, 9.3 mmol) were dissolved in DCM (10.0 mL). The reaction mixture was stirred at 40°C for 3 hours. TLC analysis indicated complete consumption of compound 20. The reaction mixture was poured into water and extracted with EA. The organic phase was washed with brine, dried over Na₂SO₄, and concentrated. Compound 21 was purified by column chromatography (DCM: MeOH=150:0-20:1). The H1-NMR spectrum is shown in Figure 17.

### Example 3: Preparation of LNPs@mRNA

Based on the compounds obtained in Examples 1 and 2 (Compounds 7 to 13, Compounds 15 to 19, and Compound 21), the inventors constructed an mRNA delivery system LNPs@mRNA (lipid nanoparticles loaded with mRNA) using commercially available MC3, SM-102, and ALC-0315 as control lipids. The formulation properties of LNPs@mRNA, including particle size, zeta potential, encapsulation efficiency, mRNA integrity, TEM, etc., were investigated to evaluate its formulation characteristics. Hereinafter, the compounds of the present invention, MC3, SM-102, and ALC-0315 are collectively referred to as ionizable lipids.

Preparation Method of LNPs@mRNA:
(1) Solution Preparation: Dissolve ionizable lipids, DOPE (or DSPC), cholesterol (Chol), and DMG-PEG2000 in anhydrous ethanol to obtain an ionizable lipid solution. The concentration of ionizable lipids in the solution is 10 mg/mL. The molar ratio of ionizable lipids, DSPC, cholesterol (Chol), and DMG-PEG2000 is 50:10:38.5:1.5. The mRNA is diluted to an appropriate concentration in 10 mM pH 6.0 PBS buffer (prepared with RNase-free water) and kept aside. Typically, mRNA sequences encoding viral antigen proteins (in this example, mRNA sequences encoding the Delta S protein of the novel coronavirus are selected to prepare LNPs@mRNA formulations for further investigation of their immune activation efficacy) or mRNA sequences encoding tumor antigens, therapeutic proteins or peptides, or gene editing tools such as Cas9 protein sequences are chosen.
(2) LNP Preparation: The ionizable lipid solution obtained in step (1) is mixed with the mRNA solution to obtain the LNP primary formulation. The mass ratio of ionizable lipids to mRNA is controlled at 8:1. The mixing process is conducted in a microfluidic device with the following parameters: the volume ratio of ethanol phase to water phase is 1:4, and the flow rate is 9 mL/min.
(3) Ultrafiltration: The LNP primary formulation is diluted 25-fold with PBS buffer and then ultrafiltered using a filtration cup to the initial volume to obtain the LNP final formulation. During the ultrafiltration process, ethanol in the primary formulation is removed, resulting in different LNPs@mRNA drugs, referred to as Compounds 7 to 13, Compounds 15 to 19, Compound 21, MC3, SM-102, and ALC-0315 drugs, respectively. The ultrafiltration parameters include a 100 kDa filter membrane, an air pressure of 0.2 MPa, and a rotation speed of 100-200 rpm.

Investigation of Formulation Properties of LNPs@mRNA:
Particle Size and Zeta Potential Measurement: An appropriate amount of LNPs@mRNA formulation is diluted 10-fold with purified water, and their particle size, size distribution, and ζ potential are measured using a Malvern nano particle size and zeta potential analyzer to assess formulation properties (n=3, i.e., each formulation is tested three times).

Transmission Electron Microscopy (TEM) Characterization: LNPs@mRNA is diluted with purified water to achieve a nanoparticle mass concentration of 2 mg/mL. Carefully, the diluted solution is dropped onto a TEM-specific copper grid and allowed to stand for 2 minutes. Excess liquid is removed using filter paper, and then 2% phosphotungstic acid negative staining is applied for 3 minutes. After removing excess staining solution, the sample is dried with an ear bulb blower and then subjected to examination and photography.

Encapsulation Efficiency Detection: The encapsulation efficiency is measured using the Quant-iT^{™} RiboGreen^{™} assay kit.

mRNA Integrity Detection: To further investigate the protective effect of the LNPs prepared in this invention on mRNA, the integrity of mRNA in the formulation is characterized and compared with samples of mRNA solution without ionizable lipids added before undergoing the formulation process.

The LNPs prepared with different ionizable lipids according to the present invention exhibit comparable particle sizes, with average sizes around 100 nm, Di(90) within 500 nm, and PDI below 0.3, indicating a uniform particle size distribution. The zeta potential of the formulations is approximately 5 mV.

The encapsulation efficiency results show that LNPs@mRNA prepared using different ionizable lipids obtained from embodiments 1 and 2 of the invention, as well as commercially available ionizable lipids, all have encapsulation efficiencies of over 80%. This indicates that the ionizable lipids provided in the present invention offer good protection for mRNA in different formulation methods.

Integrity testing results indicate that the integrity of mRNA significantly decreases after undergoing the formulation process without the addition of ionizable lipids compared to the starting mRNA solution. However, compared to the starting mRNA solution, LNPs@mRNA formulations show no significant decrease in integrity.

Overall, these experimental results demonstrate that LNPs@mRNA prepared with ionizable lipids according to the present invention exhibit excellent nanoparticle formulation properties, with encapsulation efficiencies above 80% and good protection for mRNA.

### Test Example: Effect Validation of Compounds in the Present Invention

### 1. Evaluation of Cell Toxicity of Compounds in the Present Invention

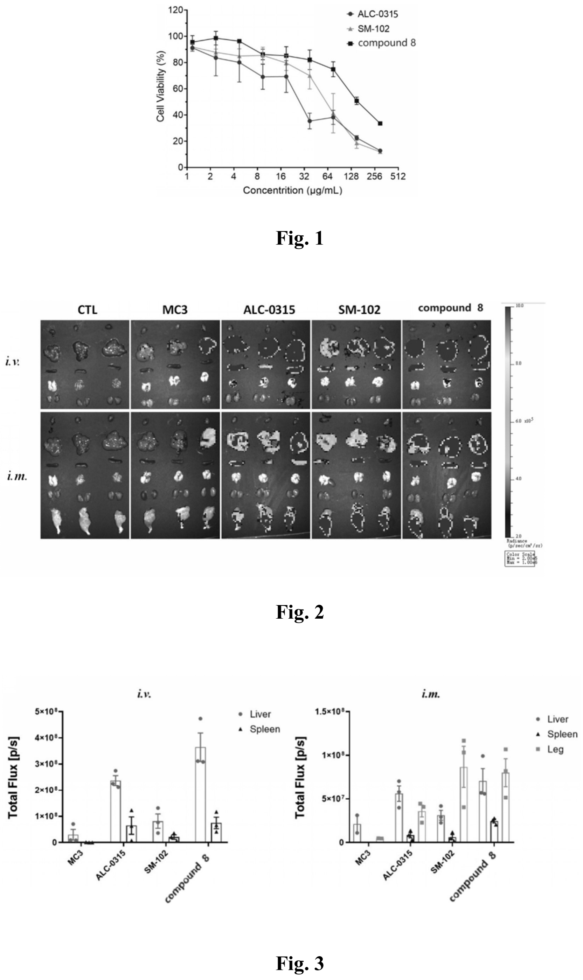

CHL cells are seeded in a 96-well culture plate at a density of 4000 cells per well and incubated overnight at 37°C in a 5% CO₂ incubator. The culture medium is then replaced with 100 µL of medium containing different concentrations of ALC-0315, SM-102, and compounds prepared according to embodiments 1 and 2 of the present invention (specific concentrations are shown in Figure 1), and cells are further incubated for 48 hours. Subsequently, 10 µL of CCK8 solution is added to each well and incubated for an additional 4 hours in the cell culture incubator. After the incubation, the absorbance at 450 nm is measured using a microplate reader, and cell viability is calculated. The IC50 values are calculated using GraphPad Prism software (version 8.01).

The results of the cell toxicity experiments are shown in Figure 1 and Table 1. Under the experimental conditions, the cell toxicity of the ionizable lipids in the present invention is lower than that of the ionizable lipid components in commercially available mRNA vaccine products, demonstrating better biocompatibility. Moreover, compared to the commercial lipid components ALC-0315 and SM-102 (with IC₅₀ values of 29.9 and 55.9, respectively), the IC₅₀ value of Compound 8 is 166.5, indicating superior performance.

**Table 1**

| Lipid materials | IC₅₀ (µg/mL) | Lipid materials | IC₅₀ (µg/mL) |
|---|---|---|---|
| ALC-0315 | 29.9 | Compound 13 | 135.8 |
| SM-102 | 55.9 | Compound 15 | 147.3 |
| Compound 7 | 125.6 | Compound 16 | 133.5 |
| Compound 8 | 166.5 | Compound 17 | 173.6 |
| Compound 9 | 152.5 | Compound 18 | 162.5 |
| Compound 10 | 152.8 | Compound 19 | 156.1 |
| Compound 11 | 137.4 | Compound 21 | 119.4 |
| Compound 12 | 194.6 | / | / |

### 2. In vivo Expression and Distribution of LNPs@mRNA in the Present Invention

Furthermore, the ability of LNPs@mRNA prepared using compounds of the present invention to deliver mRNA in vivo was investigated. The inventors chose firefly luciferase as the reporter gene to label mRNA, referred to as Fluc mRNA. Following the method outlined in Example 3, LNPs@Fluc mRNA were prepared using different ionizable lipids (Compound 8, MC3, ALC-0315, or SM-102), referred to as LNP@Fluc mRNA formulation, MC3@Fluc mRNA, ALC-0315@Fluc mRNA, and SM-102@Fluc mRNA, respectively. These formulations were administered via intravenous injection (Step 2.1 of this example) and intramuscular injection (Step 2.2 of this example) to investigate the ability of LNPs@mRNA formulations prepared using compounds of the present invention to express mRNA in vivo and the distribution of expression.

### 2.1. Investigation of In Vivo Expression via Intravenous Injection:

The formulations LNPs@Fluc mRNA (referred to as Compound 8 when ionizable lipid Compound 8 was used), MC3@Fluc mRNA, ALC-0315@Fluc mRNA, and SM-102@Fluc mRNA, abbreviated as follows, were prepared as described above.

Each formulation's mRNA concentration was adjusted to 0.05 mg/mL using PBS solution, and the osmolarity of each formulation was adjusted to isotonic. Each NIH mouse received a tail vein injection of 200 µL of the formulation, corresponding to 10 µg FLuc mRNA per mouse, with 3 mice per group. PBS was used as the negative control (CTL). The mice were kept on a normal diet after administration. Six hours after administration, 200 µL of substrate solution (15 mg/mL luciferin potassium salt) was injected intraperitoneally. Timing commenced after substrate injection, and euthanasia was performed 10 minutes later. The heart, liver, spleen, lungs, and kidneys were rapidly dissected, and the bioluminescence intensity of each excised organ was measured using an IVIS instrument with an exposure time of 60 s. Total flux of each organ was calculated after imaging. The results are shown in Figure 2 (i.v.) and Figure 3 (i.v.).

The results of the intravenous injection route experiment for in vivo expression indicate that Compound 8 ionizable lipids have stronger mRNA expression capability compared to the three positive control materials based on the total bioluminescence emitted. Furthermore, it was observed that LNPs@mRNA prepared using lipids of the present invention exhibited certain expression levels in the spleen, which is advantageous for the development of intravenous delivery route for tumor vaccines.

### 2.2. Investigation of In Vivo Expression via Intramuscular Injection:

The formulations LNPs@Fluc mRNA (Compound 8), the positive control formulation MC3@Fluc mRNA, ALC-0315@Fluc mRNA, and SM-102@Fluc mRNA, abbreviated as follows, were prepared as described above.

Each formulation's mRNA concentration was adjusted to 0.1 mg/mL using PBS solution, and the osmolarity of each formulation was adjusted to isotonic. Each BALB/c mouse received a 200 µL intramuscular injection in the hind leg, corresponding to 20 µg FLuc mRNA per mouse, with 3 mice per group. PBS was used as the negative control. The mice were kept on a normal diet after administration. Eight hours after administration, 200 µL of substrate solution (15 mg/mL luciferin potassium salt), corresponding to 3 mg per mouse, was injected intraperitoneally. Timing commenced after substrate injection. Ten minutes later, the mice were placed in an anesthesia device and were fully anesthetized. The bioluminescence intensity of the entire body of the live mice was measured using an IVIS instrument with an exposure time of 60 s. Total flux of each organ was calculated after imaging. The results are shown in Figure 2 (i.m.) and Figure 3 (i.m.).

The results of the intramuscular injection route experiment for in vivo expression indicate that Compound 8 ionizable lipids have stronger mRNA expression capability compared to the three positive control materials based on the total bioluminescence emitted. Furthermore, it was observed that LNPs@mRNA prepared using lipids of the present invention exhibited certain expression levels in the spleen, which is advantageous for the development of intramuscular injection route for tumor vaccines.

### 3. Immunostimulatory Efficacy of LNPs@Delta S-2P mRNA:

Following the method described in Example 3, LNPs@Delta S-2P mRNA was prepared using Compound 8 and SM-102 as ionizable lipids, abbreviated as Compound 8 and SM-102, respectively. In this example, the lipid SM-102 from a commercially available product served as the positive control. Delta S-2P mRNA encodes the Delta S-2P protein, as represented by the amino acid sequence shown in SEQ ID NO:1. Delta S-2P is a protein derived from the full-length S protein of the Delta variant strain B.1.617.2, with K986P and V987P mutations. The amino acid sequence is as follows:

MFVFLVLLPLVSSQCVNLRTRTQLPPAYTNSFTRGVYYPDKVFRSSVLHSTQDLF
LPFFSNVTWFHAIHVSGTNGTKRFDNPVLPFNDGVYFASTEKSNIIRGWIFGTTLDSKT
QSLLIVNNATNVVIKVCEFQFCNDPFLDVYYHKNNKSWMESEVYSSANNCTFEYVSQ
PFLMDLEGKQGNFKNLREFVFKNIDGYFKIYSKHTPINLVRDLPQGFSALEPLVDLPIG
INITRFQTLLALHRSYLTPGDSSSGWTAGAAAYYVGYLQPRTFLLKYNENGTITDAVD
CALDPLSETKCTLKSFTVEKGIYQTSNFRVQPTESIVRFPNITNLCPFGEVFNATRFASV
YAWNRKRISNCVADYSVLYNSASFSTFKCYGVSPTKLNDLCFTNVYADSFVIRGDEVR
QIAPGQTGKIADYNYKLPDDFTGCVIAWNSNNLDSKVGGNYNYRYRLFRKSNLKPFE
RDISTEIYQAGSKPCNGVEGFNCYFPLQSYGFQPTNGVGYQPYRVVVLSFELLHAPAT
VCGPKKSTNLVKNKCVNFNFNGLTGTGVLTESNKKFLPFQQFGRDIADTTDAVRDPQ
TLEILDITPCSFGGVSVITPGTNTSNQVAVLYQGVNCTEVPVAIHADQLTPTWRVYSTG
SNVFQTRAGCLIGAEHVNNSYECDIPIGAGICASYQTQTNSRRRARSVASQSIIAYTMS
LGAENSVAYSNNSIAIPTNFTISVTTEILPVSMTKTSVDCTMYICGDSTECSNLLLQYGS

FCTQLNRALTGIAVEQDKNTQEVFAQVKQIYKTPPIKDFGGFNFSQILPDPSKPSKRSFI
EDLLFNKVTLADAGFIKQYGDCLGDIAARDLICAQKFNGLTVLPPLLTDEMIAQYTSA
LLAGTITSGWTFGAGAALQIPFAMQMAYRFNGIGVTQNVLYENQKLIANQFNSAIGKI
QDSLSSTASALGKLQNVVNQNAQALNTLVKQLSSNFGAISSVLNDILSRLDPPEAEVQ
IDRLITGRLQSLQTYVTQQLIRAAEIRASANLAATKMSECVLGQSKRVDFCGKGYHL
MSFPQSAPHGVVFLHVTYVPAQEKNFTTAPAICHDGKAHFPREGVFVSNGTHWFVTQ
RNFYEPQIITTDNTFVSGNCDVVIGIVNNTVYDPLQPELDSFKEELDKYFKNHTSPDV
DLGDISGINASVVNIQKEIDRLNEVAKNLNESLIDLQELGKYEQYIKWPWYIWLGFIA
GLIAIVMVTIMLCCMTSCCSCLKGCCSCGSCCKFDEDDSEPVLKGVKLHYT(SEQ ID
NO:1).

6-8 week-old male Balb/c mice were divided into two groups. Each group of mice was immunized with either Compound 8 or SM-102 prepared as described above. Three different dosages were administered to each group, with three mice per dosage gradient. PBS was used as the negative control. The immunization was conducted via intramuscular injection, with a total of two administrations administered 14 days apart. Serum samples were collected from the mice 14 and 28 days after the initial immunization, and the antibody titers against the RBD of the wild-type Delta strain S protein were detected using ELISA. The experimental results are shown in Figure 4 and Table 2. Geometric mean titers (GMT) of the compounds were calculated.

The results demonstrate that the mRNA vaccine prepared with Compound 8 effectively activates the immune system, resulting in the production of specific antibodies with antibody titers reaching up to 10^6. Furthermore, compared to the positive control, the immunostimulatory effect of the mRNA vaccine prepared with Compound 8 exhibits significant advantages, with the GMT increasing by a factor of at least 2-fold, and up to 14-fold in some cases.

**Table 2**

| Time | Dose (µg) | Lipid materials | GMT | Compound 8 GMT/ SM-102 GMT |
|---|---|---|---|---|
| Day 14 | 0.1 | SM-102 | 712.7 | 6.3 |
| | | Compound 8 | 4525.5 | |
| | 1 | SM-102 | 2262.7 | 14.3 |
| | | Compound 8 | 32254.0 | |
| | 5 | SM-102 | 18101.9 | 4.5 |
| | | Compound 8 | 81274.9 | |
| Day 28 | 0.1 | SM-102 | 18101.9 | 3.6 |
| | | Compound 8 | 64508.0 | |
| | 1 | SM-102 | 81274.9 | 4.0 |
| | | Compound 8 | 325099.7 | |
| | 5 | SM-102 | 325099.7 | 2.5 |
| | | Compound 8 | 819200.0 | |

Further investigation was conducted to assess the immunostimulatory effect (antibody titers) of LNPs@Delta S-2P mRNA prepared using various compounds of the present invention.

Male Balb/c mice aged 6-8 weeks were immunized via intramuscular injection, with a total of two administrations administered 14 days apart. The dosage administered each time was 1 µg, and serum samples were collected from the mice 28 days after the initial immunization. The antibody titers against the RBD of the wild-type Delta strain S protein were detected using ELISA, with the lipid SM-102 from commercially available products used as the positive control. Geometric mean titers (GMT) for each compound were calculated, and the experimental results are shown in Table 3. In this study, the compounds prepared according to Examples 1 and 2 were used as lipid materials for the preparation of LNPs@Delta S-2P mRNA, referred to as Compound 7-Compound 13, Compound 15-Compound 19, and Compound 20, respectively. The specific preparation methods can be found in Example 3, and the Delta S-2P mRNA encodes the amino acid sequence of the Delta S-2P protein as shown in SEQ ID NO: 1.

The results demonstrate that mRNA vaccines prepared using lipid materials from Examples 1-2 (Compound 7-Compound 13, Compound 15-Compound 19, and Compound 20) can effectively activate the immune system, resulting in the production of specific antibodies with antibody titers reaching up to 10^6. Furthermore, compared to the positive control, the immunostimulatory effect of mRNA vaccines prepared using lipid materials from the present invention exhibits significant advantages, with the GMT increasing by a factor of at least 2-fold.

**Table 3**

| Lipid aterials | GMT | Lipid aterials | GMT |
|---|---|---|---|
| SM-102 | 81,274.9 | Compound 13 | 210,944.0 |
| Compound 7 | 341,832.9 | Compound 15 | 400,363.5 |
| Compound 8 | 325,099.7 | Compound 16 | 244,238.6 |
| Compound 9 | 452,334.1 | Compound 17 | 299,707.3 |
| Compound 10 | 299,812.1 | Compound 18 | 244,450.7 |
| Compound 11 | 473,681.3 | Compound 19 | 212,301.7 |
| Compound 12 | 449,359.1 | Compound 21 | 208,203.5 |

In the description of this specification, reference terms such as "an embodiment," "some embodiments," "example," "specific example," or "some examples" imply that the specific features, structures, materials, or characteristics described in conjunction with that embodiment or example are included in at least one embodiment or example of the present invention. In this specification, the illustrative statements regarding the above terms need not necessarily apply to the same embodiment or example. Furthermore, specific features, structures, materials, or characteristics described can be combined in any one or more embodiments or examples in an appropriate manner. Additionally, in situations where they are not mutually contradictory, those skilled in the art can combine and integrate different embodiments or examples and their features as described in this specification.

Although embodiments of the present invention have been shown and described above, it is understood that these embodiments are exemplary and should not be construed as limiting the present invention. Those skilled in the art can make variations, modifications, substitutions, and alterations to the above embodiments within the scope of the present invention.

## Claims

1. A compound, which is the compound represented by formula (I) or a stereoisomer, tautomer, solvate, or pharmaceutically acceptable salt of the compound represented by formula (I): 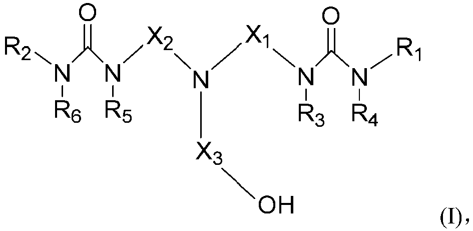
wherein X₁, X₂, and X₃ are each independently selected from optionally substituted C₁-C₁₅ alkyl groups;
R₁ and R₂ are each independently selected from optionally substituted C1-C40 alkyl, optionally substituted C₁-C₄₀ heteroalkyl, optionally substituted C₂-C₄₀ alkenyl, optionally substituted C₂-C₄₀ heteroalkenyl, optionally substituted C₂-C₄₀ alkynyl, or optionally substituted C₂-C₂₄ heteroalkynyl;
R₃, R₄, R₅, and R₆ are each independently selected from H, halogen, or optionally substituted C₁-C₃ alkyl;
wherein the substituents are independently selected from one or more of halogen, -OH, - SH, -NH₂, -NO₂, cyanide, or C₁-C₃ alkyl.

2. The compound according to claim 1, **characterized in that** X₁ and X₂ are each independently a C₄-C₁₂ alkyl group;
optionally, X₃ is a C₂-C₈ alkyl group;
optionally, X₁ is a C₄ alkyl group, X₂ is a C₄ alkyl group, and X₃ is a C₂ alkyl group, not concurrently.

3. The compound according to claim 1, **characterized in that** R₃, R₄, R₅, and R₆ are each independently H or a halogen.

4. The compound according to claim 1, **characterized in that** R1 and R₂ each independently have a structure represented by formula (II): 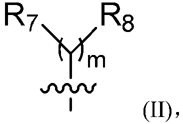
wherein m is an integer from 1 to 10;
R₇ and R₈ are each independently selected from H, optionally substituted C₁-C₂₀ alkyl, optionally substituted C₁-C₂₀ heteroalkyl, optionally substituted C₂-C₂₀ alkenyl, optionally substituted C₂-C₂₀ heteroalkenyl, optionally substituted C₂-C₂₀ alkynyl, or optionally substituted C₂-C₂₀ heteroalkynyl, where the substituents are independently selected from one or more of halogen, -OH, -SH, -NH₂, -NO₂, cyanide, or C₁-C₃ alkyl.

5. The compound according to claim 4, **characterized in that** m is 2, 3, or 4;
optionally, R₇ and R8 are each independently selected from H, optionally substituted C₄-C₁₈ alkyl, optionally substituted C₄-C₁₈ heteroalkyl, optionally substituted C₄-C₁₈ alkenyl, or optionally substituted C₄-C₁₈ heteroalkenyl, where the substituents are independently selected from one or more of halogen, -OH, -SH, -NH₂, -NO₂, cyanide, or Cl-C₃ alkyl.

6. The compound according to claim 4 or 5, **characterized in that** R1 and R₂ each independently have a structure represented by formula (III): 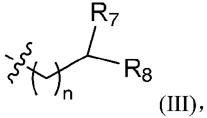
wherein n is 1 or 2;
R₇ and R₈ are each independently selected from H, optionally substituted C₁-C₂₀ alkyl, optionally substituted C₁-C₂₀ heteroalkyl, optionally substituted C₂-C₂₀ alkenyl, optionally substituted C₂-C₂₀ heteroalkenyl, optionally substituted C₂-C₂₀ alkynyl, or optionally substituted C₂-C₂₀ heteroalkynyl, wherein the substituents are independently selected from one or more of halogen, -OH, -SH, -NH2, -NO₂, cyanide, or C₁-C₃ alkyl;
Optionally, R₇ and R8 are each independently selected from optionally substituted C₄-C₁₈ alkyl, optionally substituted C₄-C₁₈ heteroalkyl, optionally substituted C₄-C₁₈ alkenyl, or optionally substituted C₄-C₁₈ heteroalkenyl, where the substituents are independently selected from one or more of halogen, -OH, -SH, -NH₂, -NO₂, cyanide, or C₁-C₃ alkyl.

7. The compound according to claim 6, **characterized in that** R₁ and R₂ each independently have at least one of the following structures:

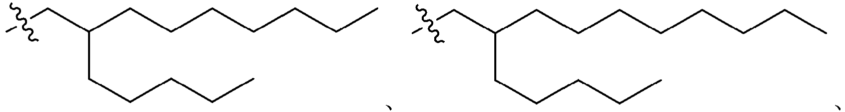

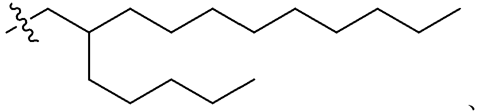

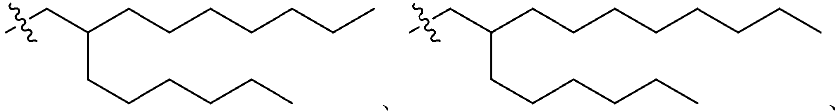

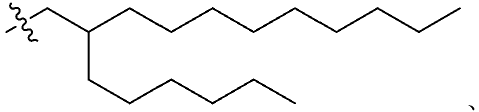

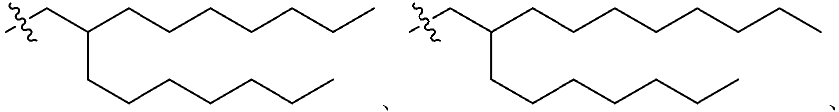

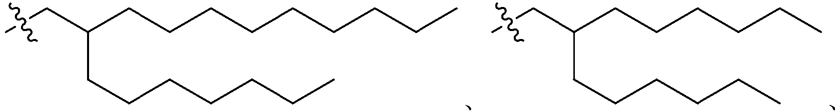

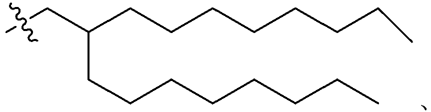

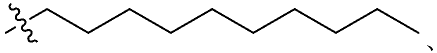

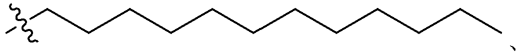

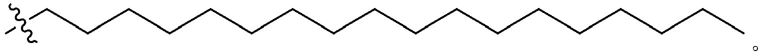

8. According to the compound described in claim 1, it is **characterized in that** the said compound has at least one of the following structures:

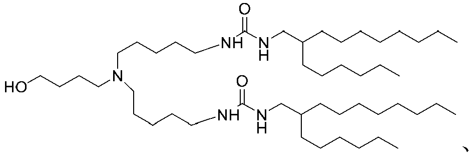

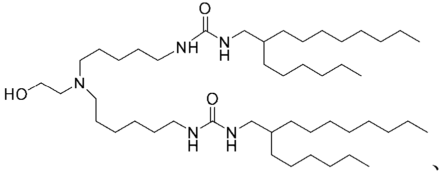

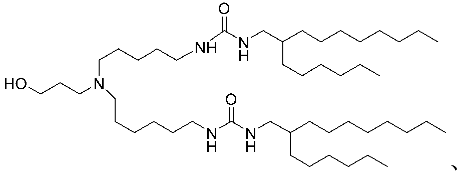

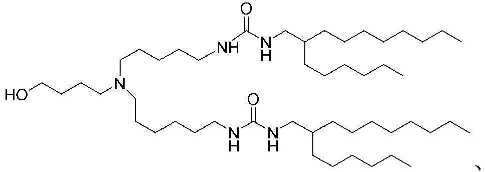

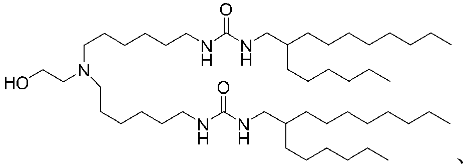

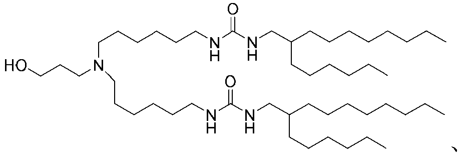

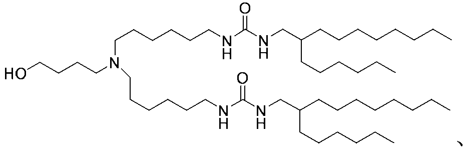

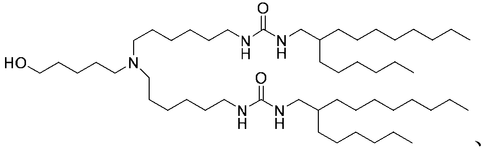

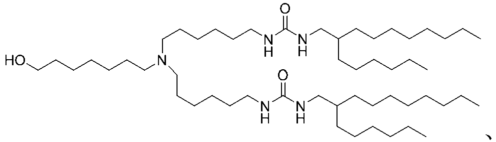

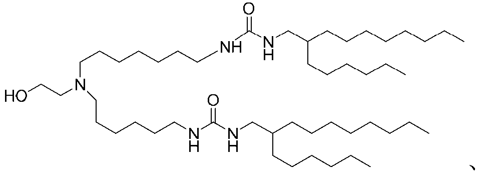

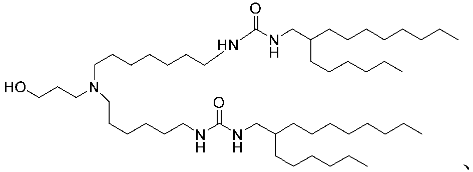

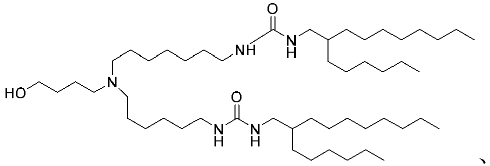

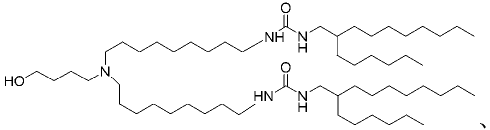

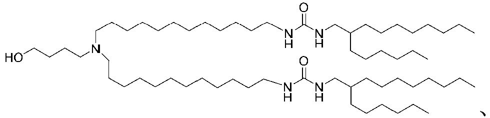

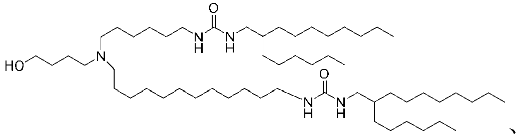

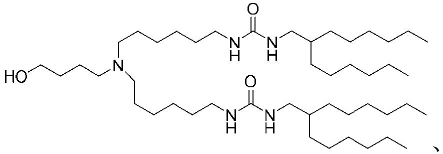

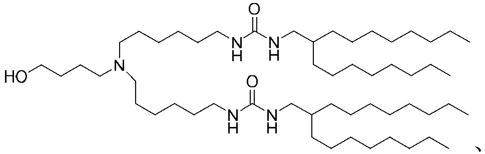

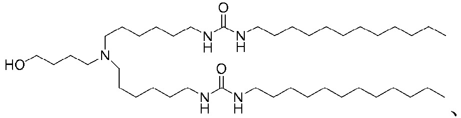

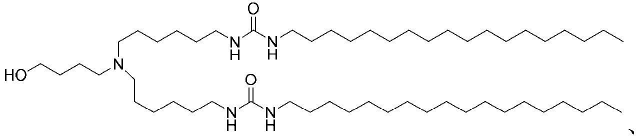

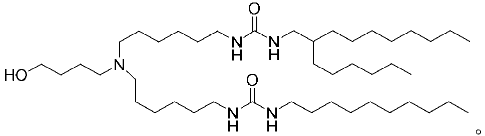

9. The use of the compound according to any one of claims 1 to 8 in the preparation of liposomes, drug carriers, or complexes.

10. A liposome, **characterized in that** it includes: the compound according to any one of claims 1 to 8.

11. The liposome according to claim 10, **characterized in that** it further comprises:
At least one of steroids, neutral lipids, and PEG-lipids;
optionally, said neutral lipids include at least one selected from 1,2-distearoyl-sn-glycero-3-phosphocholine (DSPC), 1,2-dipalmitoyl-sn-glycero-3-phosphocholine (DPPC), 1,2-dipalmitoyl-sn-glycerol-3-phosphate ethanolamine (DPPE), 1,2-dimyristoyl-sn-glycero-3-phosphocholine (DMPC), 1,2-dimyristoyl-sn-glycerol-3-phosphate ethanolamine (DMPE), 1-palmitoyl-2-oleoyl-sn-glycero-3-phosphocholine (POPC), 1,2-dioleoyl-sn-glycero-3-phosphocholine (DOPC), 1,2-dioleoyl-sn-glycerol-3-phosphate-(1'-rac-glycerol) (DOPG), 1,2-dioleoyl-sn-glycero-3-phosphate ethanolamine (DOPE), and sphingomyelin (SM);
optionally, said PEG-lipids include at least one selected from 2-[(polyethylene glycol)-2000]-N,N-ditetradecylacetamide (ALC-0159), methoxy polyethylene glycol-dimyristoyl glycerol (PEG-DMG), polyethylene glycol-distearoyl phosphatidylethanolamine (PEG-DSPE), PEG-disterol glycerol (PEG-DSG), PEG-dipalmitoyl, PEG-dioleyl, PEG-distearoyl, PEG-diacyl glyceramide (PEG-DAG), PEG-dipalmitoyl phosphatidylethanolamine (PEG-DPPE), PEG-phosphatidylethanolamine (PEG-PE), PEG-succinate diacyl glycerol (PEG-S-DAG), PEG-ceramide (PEG-cer), PEG-dialkoxypropyl carbamate, and PEG-1,2-dimyristoyl oxypropyl-3-amine (PEG-c-DMA);
preferably, said steroids include at least one selected from cholesterol, coprosterol, ergosterol, ergocalciferol, vegetable sterol, sitosterol, and rapeseed sterol, preferably cholesterol.

12. The liposome according to claim 11, **characterized in that** the compound: neutral lipid: steroid: PEG-lipid molar ratio is (20~80):(5~50):(10~60):(0.01~10);
preferably, the compound:neutral lipid:steroid:PEG-lipid molar ratio is (40~60):(5~10):(30~50):(0.5~5)_{∘}

13. A liposome, **characterized in that** it comprises the compound according to any one of claims 1 to 8, neutral lipids, cholesterol, and PEG-lipids, wherein the neutral lipids include at least one selected from 1,2-dioleoyl-sn-glycerol-3-phosphate ethanolamine (DOPE) and/or 1,2-dioleoyl-sn-glycerol-3-phosphocholine (DOPC), and the PEG-lipid is methoxy polyethylene glycol-dimyristoyl glycerol (PEG-DMG), and the compound, neutral lipid, cholesterol, and PEG-lipid molar ratio is (40~60):(5~10):(30~50):(0.5~5)_{∘}

14. A drug carrier, **characterized in that** it includes the compound according to any one of claims 1 to 8 or the liposome according to claims 10 to 13.

15. A complex, **characterized in that** it includes:
the compound according to any one of claims 1 to 8, the liposome according to claims 10 to 13, or the drug carrier according to claim 14; and
a biologically active ingredient.

16. The complex according to claim 15, **characterized in that** the biologically active ingredient includes at least one selected from DNA molecules, RNA molecules, proteins, peptides, and small molecule drugs;
optionally, the biologically active ingredient carries a negative charge or is hydrophobic;
preferably, the biologically active ingredient is nucleic acid;
more preferably, the biologically active ingredient is antisense RNA and messenger RNA.

17. A pharmaceutical composition, **characterized in that** it includes:
the compound according to any one of claims 1 to 8, the liposome according to claims 10 to 13, the drug carrier according to claim 14, or the complex according to claims 15 to 16;
optionally pharmaceutically acceptable excipients.

18. The use of the compound according to any one of claims 1 to 8, the liposome according to claims 10 to 13, the drug carrier according to claim 14, the complex according to claims 15 to 16, or the pharmaceutical composition according to claim 17 in the preparation of a drug, wherein the drug is used to target at least one of the heart, liver, spleen, lung, and kidney;
preferably, the drug is used to target the spleen.

19. The use of the compound according to any one of claims 1 to 8, the liposome according to claims 10 to 13, the drug carrier according to claim 14, the complex according to claims 15 to 16, or the pharmaceutical composition according to claim 17 in the preparation of a drug, wherein the drug is used for the treatment or prevention of diseases;
optionally, the diseases include selected from tumors or tumor-related diseases, virus-induced related diseases.
